# EUROPÄISCHE PATENTANMELDUNG

(11) **EP 4 006 164 A1**
(43) Veröffentlichungstag der Anmeldung: **01.06.2022**
(21) Anmeldenummer: 20210409.7
(22) Anmeldetag: 27.11.2020
(51) Int. Cl.: C12P 23/00, C07K 19/00, A61K 31/7076

(54) **VERFAHREN ZUR HERSTELLUNG EINES PFLANZENINHALTSSTOFFES DURCH OPTOGENETISCHE KONTROLLE VON ZELLZYKLUSREGULATOREN**

(71) Anmelder: Philipps-Universität Marburg, 35037 Marburg (DE)
(72) Erfinder: Essen, Lars-Oliver, 35043 Marburg (DE); Taxis, Christof, 35392 Gießen (DE); Bezold, Filipp, 35037 Marburg (DE); Scheffer, Johannes, 35037 Marburg (DE)
(74) Vertreter: Stumpf, Peter

(57) **Zusammenfassung**

Die Erfindung betrifft ein Verfahren zur Herstellung eines pflanzlichen oder pilzlichen Inhaltsstoffes in eukaryotischen Zellen durch optogenetische Kontrolle von mindestens einem Zellzyklusregulator. Dabei werden optogenetische Werkzeuge zur gezielten Transkriptionskontrolle in Hefe (*Saccharomyces cerevisiae*) genutzt, um eine Veränderung des Metaboloms herbeizuführen. Das hat den Vorteil die Ausbeute bei der heterologen Biosynthese von Stoffen insbesondere Pflanzeninhaltsstoffen wie z.B. β-Carotin und Gibberellin oder pilzlichen Inhaltsstoffen wie z.B. Cordycepin zu steigern.

SEQUENCE LISTING

<110> Philipps-Universität Marburg

<120> Verfahren zur Herstellung eines Pflanzeninhaltsstoffes durch
optogenetische Kontrolle von Zellzyklusregulatoren

<130> TM1061

<160> 5

<170> BiSSAP 1.3.6

<210> 1
<211> 1066
<212> PRT
<213> Artificial Sequence

<220>
<223> Zellzyklusregulator Cdc48-psd1

<400> 1
Met Gly Glu Glu His Lys Pro Leu Leu Asp Ala Ser Gly Val Asp Pro
1               5                   10                  15
Arg Glu Glu Asp Lys Thr Ala Thr Ala Ile Leu Arg Arg Lys Lys Lys
            20                  25                  30
Asp Asn Met Leu Leu Val Asp Asp Ala Ile Asn Asp Asp Asn Ser Val
        35                  40                  45
Ile Ala Ile Asn Ser Asn Thr Met Asp Lys Leu Glu Leu Phe Arg Gly
    50                  55                  60
Asp Thr Val Leu Val Lys Gly Lys Lys Arg Lys Asp Thr Val Leu Ile
65                  70                  75                  80
Val Leu Ile Asp Asp Glu Leu Glu Asp Gly Ala Cys Arg Ile Asn Arg
                85                  90                  95
Val Val Arg Asn Asn Leu Arg Ile Arg Leu Gly Asp Leu Val Thr Ile
            100                 105                 110
His Pro Cys Pro Asp Ile Lys Tyr Ala Thr Arg Ile Ser Val Leu Pro
        115                 120                 125
Ile Ala Asp Thr Ile Glu Gly Ile Thr Gly Asn Leu Phe Asp Val Phe
    130                 135                 140
Leu Lys Pro Tyr Phe Val Glu Ala Tyr Arg Pro Val Arg Lys Gly Asp
145                 150                 155                 160
His Phe Val Val Arg Gly Gly Met Arg Gln Val Glu Phe Lys Val Val
                165                 170                 175
Asp Val Glu Pro Glu Glu Tyr Ala Val Val Ala Gln Asp Thr Ile Ile
            180                 185                 190
His Trp Glu Gly Glu Pro Ile Asn Arg Glu Asp Glu Glu Asn Asn Met
        195                 200                 205
Asn Glu Val Gly Tyr Asp Asp Ile Gly Gly Cys Arg Lys Gln Met Ala
    210                 215                 220
Gln Ile Arg Glu Met Val Glu Leu Pro Leu Arg His Pro Gln Leu Phe
225                 230                 235                 240
Lys Ala Ile Gly Ile Lys Pro Pro Arg Gly Val Leu Met Tyr Gly Pro
                245                 250                 255
Pro Gly Thr Gly Lys Thr Leu Met Ala Arg Ala Val Ala Asn Glu Thr
            260                 265                 270
Gly Ala Phe Phe Phe Leu Ile Asn Gly Pro Glu Val Met Ser Lys Met
        275                 280                 285
Ala Gly Glu Ser Glu Ser Asn Leu Arg Lys Ala Phe Glu Glu Ala Glu
    290                 295                 300
Lys Asn Ala Pro Ala Ile Ile Phe Ile Asp Glu Ile Asp Ser Ile Ala

305                 310                 315                 320
Pro Lys Arg Asp Lys Thr Asn Gly Glu Val Glu Arg Arg Val Val Ser
                325                 330                 335
Gln Leu Leu Thr Leu Met Asp Gly Met Lys Ala Arg Ser Asn Val Val
            340                 345                 350
Val Ile Ala Ala Thr Asn Arg Pro Asn Ser Ile Asp Pro Ala Leu Arg
        355                 360                 365
Arg Phe Gly Arg Phe Asp Arg Glu Val Asp Ile Gly Ile Pro Asp Ala
    370                 375                 380
Thr Gly Arg Leu Glu Val Leu Arg Ile His Thr Lys Asn Met Lys Leu
385                 390                 395                 400
Ala Asp Asp Val Asp Leu Glu Ala Leu Ala Ala Glu Thr His Gly Tyr
                405                 410                 415
Val Gly Ala Asp Ile Ala Ser Leu Cys Ser Glu Ala Ala Met Gln Gln
            420                 425                 430
Ile Arg Glu Lys Met Asp Leu Ile Asp Leu Asp Glu Asp Glu Ile Asp
        435                 440                 445
Ala Glu Val Leu Asp Ser Leu Gly Val Thr Met Asp Asn Phe Arg Phe
    450                 455                 460
Ala Leu Gly Asn Ser Asn Pro Ser Ala Leu Arg Glu Thr Val Val Glu
465                 470                 475                 480
Ser Val Asn Val Thr Trp Asp Asp Val Gly Gly Leu Asp Glu Ile Lys
                485                 490                 495
Glu Glu Leu Lys Glu Thr Val Glu Tyr Pro Val Leu His Pro Asp Gln
            500                 505                 510
Tyr Thr Lys Phe Gly Leu Ser Pro Ser Lys Gly Val Leu Phe Tyr Gly
        515                 520                 525
Pro Pro Gly Thr Gly Lys Thr Leu Leu Ala Lys Ala Val Ala Thr Glu
    530                 535                 540
Val Ser Ala Asn Phe Ile Ser Val Lys Gly Pro Glu Leu Leu Ser Met
545                 550                 555                 560
Trp Tyr Gly Glu Ser Glu Ser Asn Ile Arg Asp Ile Phe Asp Lys Ala
                565                 570                 575
Arg Ala Ala Ala Pro Thr Val Val Phe Leu Asp Glu Leu Asp Ser Ile
            580                 585                 590
Ala Lys Ala Arg Gly Gly Ser Leu Gly Asp Ala Gly Gly Ala Ser Asp
        595                 600                 605
Arg Val Val Asn Gln Leu Leu Thr Glu Met Asp Gly Met Asn Ala Lys
    610                 615                 620
Lys Asn Val Phe Val Ile Gly Ala Thr Asn Arg Pro Asp Gln Ile Asp
625                 630                 635                 640
Pro Ala Ile Leu Arg Pro Gly Arg Leu Asp Gln Leu Ile Tyr Val Pro
                645                 650                 655
Leu Pro Asp Glu Asn Ala Arg Leu Ser Ile Leu Asn Ala Gln Leu Arg
            660                 665                 670
Lys Thr Pro Leu Glu Pro Gly Leu Glu Leu Thr Ala Ile Ala Lys Ala
        675                 680                 685
Thr Gln Gly Phe Ser Gly Ala Asp Leu Leu Tyr Ile Val Gln Arg Ala
    690                 695                 700
Ala Lys Tyr Ala Ile Lys Asp Ser Ile Glu Ala His Arg Gln His Glu
705                 710                 715                 720
Ala Glu Lys Glu Val Lys Val Glu Gly Glu Asp Val Glu Met Thr Asp
                725                 730                 735
Glu Gly Ala Lys Ala Glu Gln Glu Pro Glu Val Asp Pro Val Pro Tyr
            740                 745                 750
Ile Thr Lys Glu His Phe Ala Glu Ala Met Lys Thr Ala Lys Arg Ser
        755                 760                 765
Val Ser Asp Ala Glu Leu Arg Arg Tyr Glu Ala Tyr Ser Gln Gln Met
    770                 775                 780
Lys Ala Ser Arg Gly Gln Phe Ser Asn Phe Asn Phe Asn Asp Ala Pro
785                 790                 795                 800
Leu Gly Thr Thr Ala Thr Asp Asn Ala Asn Ser Asn Asn Ser Ala Pro
                805                 810                 815

Ser Gly Ala Gly Ala Ala Phe Gly Ser Asn Ala Glu Glu Asp Asp Asp
            820                 825                 830
Leu Tyr Ser Arg Thr Leu Gln Val Asp Gly Ser Ser Arg Gly Glu Gln
        835                 840                 845
Lys Leu Ile Ser Glu Glu Asp Leu Asn Gly Glu Gln Lys Leu Ile Ser
    850                 855                 860
Glu Glu Asp Leu Asn Gly Glu Gln Lys Leu Ile Ser Glu Glu Asp Leu
865                 870                 875                 880
Asn Gly Ser Ser Arg Val Met Arg Lys Gly Ile Asp Leu Ala Thr Thr
                885                 890                 895
Leu Glu Arg Ile Glu Lys Asn Phe Val Ile Thr Asp Pro Arg Leu Pro
            900                 905                 910
Asp Asn Pro Ile Ile Phe Ala Ser Asp Ser Phe Leu Glu Leu Thr Glu
        915                 920                 925
Tyr Ser Arg Glu Glu Ile Leu Gly Arg Asn Cys Arg Phe Leu Gln Gly
    930                 935                 940
Pro Glu Thr Asp Leu Thr Thr Val Lys Lys Ile Arg Asn Ala Ile Asp
945                 950                 955                 960
Asn Gln Thr Glu Val Thr Val Gln Leu Ile Asn Tyr Thr Lys Ser Gly
                965                 970                 975
Lys Lys Phe Trp Asn Ile Phe His Leu Gln Pro Met Arg Asp Gln Lys
            980                 985                 990
Gly Glu Val Gln Tyr Phe Ile Gly Val Gln Leu Asp Gly Ser Lys His
        995                 1000                1005
Val Glu Pro Val Arg Asn Val Ile Glu Glu Thr Ala Val Lys Glu Gly
    1010                1015                1020
Glu Asp Leu Val Lys Lys Thr Ala Val Asn Ile Asp Glu Ala Val Arg
1025                1030                1035                1040
Glu Leu Pro Met Ser Cys Ala Gln Glu Ser Ile Thr Ser Leu Tyr Lys
                1045                1050                1055
Lys Ala Gly Ser Glu Asn Leu Tyr Phe Gln
            1060                1065

<210> 2
<211> 1058
<212> PRT
<213> Artificial Sequence

<220>
<223> Zellzyklusregulator Cdc48-psd3

<400> 2
Met Gly Glu Glu His Lys Pro Leu Leu Asp Ala Ser Gly Val Asp Pro
1               5                   10                  15
Arg Glu Glu Asp Lys Thr Ala Thr Ala Ile Leu Arg Arg Lys Lys Lys
            20                  25                  30
Asp Asn Met Leu Leu Val Asp Asp Ala Ile Asn Asp Asp Asn Ser Val
        35                  40                  45
Ile Ala Ile Asn Ser Asn Thr Met Asp Lys Leu Glu Leu Phe Arg Gly
    50                  55                  60
Asp Thr Val Leu Val Lys Gly Lys Lys Arg Lys Asp Thr Val Leu Ile
65                  70                  75                  80
Val Leu Ile Asp Asp Glu Leu Glu Asp Gly Ala Cys Arg Ile Asn Arg
                85                  90                  95
Val Val Arg Asn Asn Leu Arg Ile Arg Leu Gly Asp Leu Val Thr Ile
            100                 105                 110
His Pro Cys Pro Asp Ile Lys Tyr Ala Thr Arg Ile Ser Val Leu Pro
        115                 120                 125
Ile Ala Asp Thr Ile Glu Gly Ile Thr Gly Asn Leu Phe Asp Val Phe
    130                 135                 140
Leu Lys Pro Tyr Phe Val Glu Ala Tyr Arg Pro Val Arg Lys Gly Asp
145                 150                 155                 160

His Phe Val Val Arg Gly Gly Met Arg Gln Val Glu Phe Lys Val Val
                    165                 170                 175
Asp Val Glu Pro Glu Glu Tyr Ala Val Val Ala Gln Asp Thr Ile Ile
                180                 185                 190
His Trp Glu Gly Glu Pro Ile Asn Arg Glu Asp Glu Glu Asn Asn Met
            195                 200                 205
Asn Glu Val Gly Tyr Asp Asp Ile Gly Gly Cys Arg Lys Gln Met Ala
    210                 215                 220
Gln Ile Arg Glu Met Val Glu Leu Pro Leu Arg His Pro Gln Leu Phe
225                 230                 235                 240
Lys Ala Ile Gly Ile Lys Pro Pro Arg Gly Val Leu Met Tyr Gly Pro
                245                 250                 255
Pro Gly Thr Gly Lys Thr Leu Met Ala Arg Ala Val Ala Asn Glu Thr
            260                 265                 270
Gly Ala Phe Phe Phe Leu Ile Asn Gly Pro Glu Val Met Ser Lys Met
        275                 280                 285
Ala Gly Glu Ser Glu Ser Asn Leu Arg Lys Ala Phe Glu Glu Ala Glu
    290                 295                 300
Lys Asn Ala Pro Ala Ile Ile Phe Ile Asp Glu Ile Asp Ser Ile Ala
305                 310                 315                 320
Pro Lys Arg Asp Lys Thr Asn Gly Glu Val Glu Arg Arg Val Val Ser
                325                 330                 335
Gln Leu Leu Thr Leu Met Asp Gly Met Lys Ala Arg Ser Asn Val Val
            340                 345                 350
Val Ile Ala Ala Thr Asn Arg Pro Asn Ser Ile Asp Pro Ala Leu Arg
        355                 360                 365
Arg Phe Gly Arg Phe Asp Arg Glu Val Asp Ile Gly Ile Pro Asp Ala
    370                 375                 380
Thr Gly Arg Leu Glu Val Leu Arg Ile His Thr Lys Asn Met Lys Leu
385                 390                 395                 400
Ala Asp Asp Val Asp Leu Glu Ala Leu Ala Ala Glu Thr His Gly Tyr
                405                 410                 415
Val Gly Ala Asp Ile Ala Ser Leu Cys Ser Glu Ala Ala Met Gln Gln
            420                 425                 430
Ile Arg Glu Lys Met Asp Leu Ile Asp Leu Asp Glu Asp Glu Ile Asp
        435                 440                 445
Ala Glu Val Leu Asp Ser Leu Gly Val Thr Met Asp Asn Phe Arg Phe
    450                 455                 460
Ala Leu Gly Asn Ser Asn Pro Ser Ala Leu Arg Glu Thr Val Val Glu
465                 470                 475                 480
Ser Val Asn Val Thr Trp Asp Asp Val Gly Gly Leu Asp Glu Ile Lys
                485                 490                 495
Glu Glu Leu Lys Glu Thr Val Glu Tyr Pro Val Leu His Pro Asp Gln
            500                 505                 510
Tyr Thr Lys Phe Gly Leu Ser Pro Ser Lys Gly Val Leu Phe Tyr Gly
        515                 520                 525
Pro Pro Gly Thr Gly Lys Thr Leu Leu Ala Lys Ala Val Ala Thr Glu
    530                 535                 540
Val Ser Ala Asn Phe Ile Ser Val Lys Gly Pro Glu Leu Leu Ser Met
545                 550                 555                 560
Trp Tyr Gly Glu Ser Glu Ser Asn Ile Arg Asp Ile Phe Asp Lys Ala
                565                 570                 575
Arg Ala Ala Ala Pro Thr Val Val Phe Leu Asp Glu Leu Asp Ser Ile
            580                 585                 590
Ala Lys Ala Arg Gly Gly Ser Leu Gly Asp Ala Gly Gly Ala Ser Asp
        595                 600                 605
Arg Val Val Asn Gln Leu Leu Thr Glu Met Asp Gly Met Asn Ala Lys
    610                 615                 620
Lys Asn Val Phe Val Ile Gly Ala Thr Asn Arg Pro Asp Gln Ile Asp
625                 630                 635                 640
Pro Ala Ile Leu Arg Pro Gly Arg Leu Asp Gln Leu Ile Tyr Val Pro
                645                 650                 655
Leu Pro Asp Glu Asn Ala Arg Leu Ser Ile Leu Asn Ala Gln Leu Arg

            660                 665                 670
Lys Thr Pro Leu Glu Pro Gly Leu Glu Leu Thr Ala Ile Ala Lys Ala
        675                 680                 685
Thr Gln Gly Phe Ser Gly Ala Asp Leu Leu Tyr Ile Val Gln Arg Ala
    690                 695                 700
Ala Lys Tyr Ala Ile Lys Asp Ser Ile Glu Ala His Arg Gln His Glu
705                 710                 715                 720
Ala Glu Lys Glu Val Lys Val Glu Gly Glu Asp Val Glu Met Thr Asp
                725                 730                 735
Glu Gly Ala Lys Ala Glu Gln Glu Pro Glu Val Asp Pro Val Pro Tyr
            740                 745                 750
Ile Thr Lys Glu His Phe Ala Glu Ala Met Lys Thr Ala Lys Arg Ser
        755                 760                 765
Val Ser Asp Ala Glu Leu Arg Arg Tyr Glu Ala Tyr Ser Gln Gln Met
    770                 775                 780
Lys Ala Ser Arg Gly Gln Phe Ser Asn Phe Asn Phe Asn Asp Ala Pro
785                 790                 795                 800
Leu Gly Thr Thr Ala Thr Asp Asn Ala Asn Ser Asn Asn Ser Ala Pro
                805                 810                 815
Ser Gly Ala Gly Ala Ala Phe Gly Ser Asn Ala Glu Glu Asp Asp Asp
            820                 825                 830
Leu Tyr Ser Arg Thr Leu Gln Val Asp Gly Ser Ser Arg Gly Glu Gln
        835                 840                 845
Lys Leu Ile Ser Glu Glu Asp Leu Asn Gly Glu Gln Lys Leu Ile Ser
    850                 855                 860
Glu Glu Asp Leu Asn Gly Glu Gln Lys Leu Ile Ser Glu Glu Asp Leu
865                 870                 875                 880
Asn Gly Ser Ser Arg Val Met Arg Arg Gly Ile Asp Leu Ala Thr Thr
                885                 890                 895
Leu Glu Arg Ile Glu Lys Asn Phe Val Ile Thr Asp Pro Arg Leu Pro
            900                 905                 910
Asp Asn Pro Ile Ile Phe Ala Ser Asp Ser Phe Leu Gln Leu Thr Glu
        915                 920                 925
Tyr Ser Arg Glu Glu Ile Leu Gly Arg Asn Cys Arg Phe Leu Gln Gly
    930                 935                 940
Pro Glu Thr Asp Arg Ala Thr Val Arg Lys Ile Arg Asp Ala Ile Asp
945                 950                 955                 960
Asn Gln Thr Glu Val Thr Val Gln Leu Ile Asn Tyr Thr Lys Ser Gly
                965                 970                 975
Lys Lys Phe Trp Asn Val Phe His Leu Gln Pro Met Arg Asp Tyr Lys
            980                 985                 990
Gly Asp Val Gln Tyr Phe Ile Gly Val Gln Leu Asp Gly Thr Glu Arg
        995                 1000                1005
Leu His Gly Ala Ala Glu Arg Glu Ala Val Met Leu Ile Lys Lys Thr
    1010                1015                1020
Ala Phe Gln Ile Ala Glu Ala Lys Glu Leu Pro Met Ser Cys Ala Gln
1025                1030                1035                1040
Glu Ser Ile Thr Ser Leu Tyr Lys Lys Ala Gly Ser Glu Asn Leu Tyr
                1045                1050                1055
Phe Gln

<210> 3
<211> 811
<212> PRT
<213> Artificial Sequence

<220>
<223> Zellzyklusregulator Npl4-psd1

<400> 3
Met Leu Ile Arg Phe Arg Ser Lys Asn Gly Thr His Arg Val Ser Cys

1               5                   10                  15
Gln Glu Asn Asp Leu Phe Gly Thr Val Ile Glu Lys Leu Val Gly Asn
                20                  25                  30
Leu Asp Pro Asn Ala Asp Val Asp Thr Phe Thr Val Cys Glu Lys Pro
        35                  40                  45
Gly Gln Gly Ile His Ala Val Ser Glu Leu Ala Asp Arg Thr Val Met
    50                  55                  60
Asp Leu Gly Leu Lys His Gly Asp Met Leu Ile Leu Asn Tyr Ser Asp
65                  70                  75                  80
Lys Pro Ala Asn Glu Lys Asp Gly Val Asn Val Glu Ile Gly Ser Val
                85                  90                  95
Gly Ile Asp Ser Lys Gly Ile Arg Gln His Arg Tyr Gly Pro Leu Arg
            100                 105                 110
Ile Lys Glu Leu Ala Val Asp Glu Glu Leu Glu Lys Glu Asp Gly Leu
        115                 120                 125
Ile Pro Arg Gln Lys Ser Lys Leu Cys Lys His Gly Asp Arg Gly Met
    130                 135                 140
Cys Glu Tyr Cys Ser Pro Leu Pro Pro Trp Asp Lys Glu Tyr His Glu
145                 150                 155                 160
Lys Asn Lys Ile Lys His Ile Ser Phe His Ser Tyr Leu Lys Lys Leu
                165                 170                 175
Asn Glu Asn Ala Asn Lys Lys Glu Asn Gly Ser Ser Tyr Ile Ser Pro
            180                 185                 190
Leu Ser Glu Pro Asp Phe Arg Ile Asn Lys Arg Cys His Asn Gly His
        195                 200                 205
Glu Pro Trp Pro Arg Gly Ile Cys Ser Lys Cys Gln Pro Ser Ala Ile
    210                 215                 220
Thr Leu Gln Gln Gln Glu Phe Arg Met Val Asp His Val Glu Phe Gln
225                 230                 235                 240
Lys Ser Glu Ile Ile Asn Glu Phe Ile Gln Ala Trp Arg Tyr Thr Gly
                245                 250                 255
Met Gln Arg Phe Gly Tyr Met Tyr Gly Ser Tyr Ser Lys Tyr Asp Asn
            260                 265                 270
Thr Pro Leu Gly Ile Lys Ala Val Val Glu Ala Ile Tyr Glu Pro Pro
        275                 280                 285
Gln His Asp Glu Gln Asp Gly Leu Thr Met Asp Val Glu Gln Val Lys
    290                 295                 300
Asn Glu Met Leu Gln Ile Asp Arg Gln Ala Gln Glu Met Gly Leu Ser
305                 310                 315                 320
Arg Ile Gly Leu Ile Phe Thr Asp Leu Ser Asp Ala Gly Ala Gly Asp
                325                 330                 335
Gly Ser Val Phe Cys Lys Arg His Lys Asp Ser Phe Phe Leu Ser Ser
            340                 345                 350
Leu Glu Val Ile Met Ala Ala Arg His Gln Thr Arg His Pro Asn Val
        355                 360                 365
Ser Lys Tyr Ser Glu Gln Gly Phe Phe Ser Ser Lys Phe Val Thr Cys
    370                 375                 380
Val Ile Ser Gly Asn Leu Glu Gly Glu Ile Asp Ile Ser Ser Tyr Gln
385                 390                 395                 400
Val Ser Thr Glu Ala Glu Ala Leu Val Thr Ala Asp Met Ile Ser Gly
                405                 410                 415
Ser Thr Phe Pro Ser Met Ala Tyr Ile Asn Asp Thr Thr Asp Glu Arg
            420                 425                 430
Tyr Val Pro Glu Ile Phe Tyr Met Lys Ser Asn Glu Tyr Gly Ile Thr
        435                 440                 445
Val Lys Glu Asn Ala Lys Pro Ala Phe Pro Val Asp Tyr Leu Leu Val
    450                 455                 460
Thr Leu Thr His Gly Phe Pro Asn Thr Asp Thr Glu Thr Asn Ser Lys
465                 470                 475                 480
Phe Val Ser Ser Thr Gly Phe Pro Trp Ser Asn Arg Gln Ala Met Gly
                485                 490                 495
Gln Ser Gln Asp Tyr Gln Glu Leu Lys Lys Tyr Leu Phe Asn Val Ala
            500                 505                 510

Ser Ser Gly Asp Phe Asn Leu Leu His Glu Lys Ile Ser Asn Phe His
        515                 520                 525
Leu Leu Leu Tyr Ile Asn Ser Leu Gln Ile Leu Ser Pro Asp Glu Trp
    530                 535                 540
Lys Leu Leu Ile Glu Ser Ala Val Lys Asn Glu Trp Glu Glu Ser Leu
545                 550                 555                 560
Leu Lys Leu Val Ser Ser Ala Gly Trp Gln Thr Leu Val Met Ile Leu
                565                 570                 575
Gln Glu Ser Gly Arg Thr Leu Gln Val Asp Gly Ser Ser Arg Gly Glu
            580                 585                 590
Gln Lys Leu Ile Ser Glu Glu Asp Leu Asn Gly Glu Gln Lys Leu Ile
        595                 600                 605
Ser Glu Glu Asp Leu Asn Gly Glu Gln Lys Leu Ile Ser Glu Glu Asp
    610                 615                 620
Leu Asn Gly Ser Ser Arg Val Met Arg Lys Gly Ile Asp Leu Ala Thr
625                 630                 635                 640
Thr Leu Glu Arg Ile Glu Lys Asn Phe Val Ile Thr Asp Pro Arg Leu
                645                 650                 655
Pro Asp Asn Pro Ile Ile Phe Ala Ser Asp Ser Phe Leu Glu Leu Thr
            660                 665                 670
Glu Tyr Ser Arg Glu Glu Ile Leu Gly Arg Asn Cys Arg Phe Leu Gln
        675                 680                 685
Gly Pro Glu Thr Asp Leu Thr Thr Val Lys Lys Ile Arg Asn Ala Ile
    690                 695                 700
Asp Asn Gln Thr Glu Val Thr Val Gln Leu Ile Asn Tyr Thr Lys Ser
705                 710                 715                 720
Gly Lys Lys Phe Trp Asn Ile Phe His Leu Gln Pro Met Arg Asp Gln
                725                 730                 735
Lys Gly Glu Val Gln Tyr Phe Ile Gly Val Gln Leu Asp Gly Ser Lys
            740                 745                 750
His Val Glu Pro Val Arg Asn Val Ile Glu Glu Thr Ala Val Lys Glu
        755                 760                 765
Gly Glu Asp Leu Val Lys Lys Thr Ala Val Asn Ile Asp Glu Ala Val
    770                 775                 780
Arg Glu Leu Pro Met Ser Cys Ala Gln Glu Ser Ile Thr Ser Leu Tyr
785                 790                 795                 800
Lys Lys Ala Gly Ser Glu Asn Leu Tyr Phe Gln
                805                 810

<210> 4
<211> 697
<212> PRT
<213> Artificial Sequence

<220>
<223> Zellzyklusregulator Clb2DB-psd3

<400> 4
Met Ser Asn Pro Ile Glu Asn Thr Glu Asn Ser Gln Asn Thr Ser Ser
1               5                   10                  15
Ser Arg Phe Leu Arg Asn Val Gln Thr Thr Phe Gln Lys Ser Asn Ala
            20                  25                  30
Asn Asn Pro Ala Leu Thr Asn Phe Lys Ser Thr Leu Asn Ser Val Lys
        35                  40                  45
Lys Glu Gly Ser Arg Ile Pro Gln Phe Thr Arg Glu Ser Val Ser Arg
    50                  55                  60
Ser Thr Ala Ala Gln Glu Glu Lys Arg Thr Leu Lys Glu Asn Gly Ile
65                  70                  75                  80
Gln Leu Pro Lys Asn Asn Leu Leu Asp Asp Lys Glu Asn Gln Asp Pro
                85                  90                  95
Ser Ser Gln Gln Phe Gly Ala Leu Thr Ser Ile Lys Glu Gly Arg Ala
            100                 105                 110

Glu Leu Pro Ala Asn Ile Ser Leu Gln Glu Ser Ser Ser Ala Lys Glu
        115                 120                 125
Ile Ile Gln His Asp Pro Leu Lys Gly Val Gly Ser Ser Thr Glu Val
    130                 135                 140
Val His Asn Ser Val Glu Asn Glu Lys Leu His Pro Ala Arg Ser Gln
145                 150                 155                 160
Leu Gln Val Arg Asn Thr Glu Ser Glu Thr Asp Ser Gly Lys Lys Arg
                165                 170                 175
Pro Ile Ser Thr Ile Val Glu Gln Glu Leu Pro Lys Lys Phe Lys Val
            180                 185                 190
Cys Asp Glu Asn Gly Lys Glu Glu Tyr Glu Trp Glu Asp Leu Asp Ala
        195                 200                 205
Glu Asp Val Asn Asp Pro Phe Met Val Ser Glu Tyr Val Asn Asp Ile
    210                 215                 220
Phe Glu Tyr Leu His Gln Leu Glu Val Ile Thr Leu Pro Lys Lys Glu
225                 230                 235                 240
Asp Leu Tyr Gln His Arg Asn Ile His Gln Asn Arg Asp Ile Leu Val
                245                 250                 255
Asn Trp Leu Val Lys Ile His Asn Lys Phe Gly Leu Leu Pro Glu Thr
            260                 265                 270
Leu Tyr Leu Ala Ile Asn Ile Met Asp Arg Phe Leu Gly Lys Glu Leu
        275                 280                 285
Val Gln Leu Asp Lys Leu Gln Leu Val Gly Thr Ser Cys Leu Phe Ile
    290                 295                 300
Ala Ser Lys Tyr Glu Glu Val Tyr Ser Pro Ser Ile Lys His Phe Ala
305                 310                 315                 320
Ser Glu Thr Asp Gly Ala Cys Thr Glu Asp Glu Ile Lys Glu Gly Glu
                325                 330                 335
Lys Phe Ile Leu Lys Thr Leu Lys Phe Asn Leu Asn Tyr Pro Asn Pro
            340                 345                 350
Met Asn Phe Leu Arg Arg Ile Ser Lys Ala Asp Asp Tyr Asp Ile Gln
        355                 360                 365
Ser Arg Thr Leu Ala Lys Phe Leu Leu Glu Ile Ser Leu Val Asp Phe
    370                 375                 380
Arg Phe Ile Gly Ile Leu Pro Ser Leu Cys Ala Ala Ala Ala Met Phe
385                 390                 395                 400
Met Ser Arg Lys Met Leu Gly Lys Gly Lys Trp Asp Gly Asn Leu Ile
                405                 410                 415
His Tyr Ser Gly Gly Tyr Thr Lys Glu Glu Leu Ala Pro Val Cys His
            420                 425                 430
Met Ile Met Asp Tyr Leu Val Ser Pro Ile Val His Asp Glu Phe His
        435                 440                 445
Arg Lys Tyr Gln Ser Arg Arg Phe Met Lys Ala Ser Ile Ile Ser Val
    450                 455                 460
Gln Trp Ala Leu Lys Val Arg Lys Asn Gly Tyr Asp Ile Met Thr Leu
465                 470                 475                 480
His Glu Ser Arg Gly Glu Gln Lys Leu Ile Ser Glu Glu Asp Leu Asn
                485                 490                 495
Gly Glu Gln Lys Leu Ile Ser Glu Glu Asp Leu Asn Gly Glu Gln Lys
            500                 505                 510
Leu Ile Ser Glu Glu Asp Leu Asn Gly Ser Ser Arg Val Met Arg Arg
        515                 520                 525
Gly Ile Asp Leu Ala Thr Thr Leu Glu Arg Ile Glu Lys Asn Phe Val
    530                 535                 540
Ile Thr Asp Pro Arg Leu Pro Asp Asn Pro Ile Ile Phe Ala Ser Asp
545                 550                 555                 560
Ser Phe Leu Gln Leu Thr Glu Tyr Ser Arg Glu Glu Ile Leu Gly Arg
                565                 570                 575
Asn Cys Arg Phe Leu Gln Gly Pro Glu Thr Asp Arg Ala Thr Val Arg
            580                 585                 590
Lys Ile Arg Asp Ala Ile Asp Asn Gln Thr Glu Val Thr Val Gln Leu
        595                 600                 605
Ile Asn Tyr Thr Lys Ser Gly Lys Lys Phe Trp Asn Val Phe His Leu

610                 615                 620
Gln Pro Met Arg Asp Tyr Lys Gly Asp Val Gln Tyr Phe Ile Gly Val
625                 630                 635                 640
Gln Leu Asp Gly Thr Glu Arg Leu His Gly Ala Ala Glu Arg Glu Ala
                645                 650                 655
Val Met Leu Ile Lys Lys Thr Ala Phe Gln Ile Ala Glu Ala Lys Glu
            660                 665                 670
Leu Pro Met Ser Cys Ala Gln Glu Ser Ile Thr Ser Leu Tyr Lys Lys
        675                 680                 685
Ala Gly Ser Glu Asn Leu Tyr Phe Gln
    690                 695

<210> 5
<211> 396
<212> PRT
<213> Artificial Sequence

<220>
<223> Zellzyklusregulator NSic1-psd3

<400> 5
Met Glu Gln Glu Pro Leu Gly Arg Val Asn Arg Ile Leu Phe Pro Thr
1               5                   10                  15
Gln Gln Asn Val Asp Ile Asp Ala Ala Glu Glu Glu Glu Gly Glu
            20                  25                  30
Val Leu Leu Pro Pro Ser Arg Pro Thr Ser Ala Arg Gln Leu His Leu
        35                  40                  45
Ser Leu Glu Arg Asp Glu Phe Asp Gln Thr His Arg Lys Lys Ile Ile
    50                  55                  60
Lys Asp Val Pro Gly Thr Pro Ser Asp Lys Val Ile Thr Phe Glu Leu
65                  70                  75                  80
Ala Lys Asn Trp Asn Asn Asn Ser Pro Lys Asn Asp Ala Arg Ser Gln
                85                  90                  95
Glu Ser Glu Asp Glu Glu Asp Ile Ile Ile Asn Pro Val Arg Val Gly
            100                 105                 110
Lys Asn Pro Phe Ala Ser Asp Glu Leu Val Thr Gln Glu Ile Arg Asn
        115                 120                 125
Glu Arg Lys Arg Ala Met Leu Arg Glu Asn Pro Asp Ile Glu Asp Val
    130                 135                 140
Ile Thr Tyr Val Asn Lys Lys Gly Glu Val Val Glu Lys Arg Arg Leu
145                 150                 155                 160
Thr Asp Glu Glu Lys Arg Arg Phe Lys Pro Lys Ala Leu Phe Gln Ser
                165                 170                 175
Arg Asp Gln Glu His Ser Arg Gly Glu Gln Lys Leu Ile Ser Glu Glu
            180                 185                 190
Asp Leu Asn Gly Glu Gln Lys Leu Ile Ser Glu Glu Asp Leu Asn Gly
        195                 200                 205
Glu Gln Lys Leu Ile Ser Glu Glu Asp Leu Asn Gly Ser Ser Arg Val
    210                 215                 220
Met Arg Arg Gly Ile Asp Leu Ala Thr Thr Leu Glu Arg Ile Glu Lys
225                 230                 235                 240
Asn Phe Val Ile Thr Asp Pro Arg Leu Pro Asp Asn Pro Ile Ile Phe
                245                 250                 255
Ala Ser Asp Ser Phe Leu Gln Leu Thr Glu Tyr Ser Arg Glu Glu Ile
            260                 265                 270
Leu Gly Arg Asn Cys Arg Phe Leu Gln Gly Pro Glu Thr Asp Arg Ala
        275                 280                 285
Thr Val Arg Lys Ile Arg Asp Ala Ile Asp Asn Gln Thr Glu Val Thr
    290                 295                 300
Val Gln Leu Ile Asn Tyr Thr Lys Ser Gly Lys Lys Phe Trp Asn Val
305                 310                 315                 320
Phe His Leu Gln Pro Met Arg Asp Tyr Lys Gly Asp Val Gln Tyr Phe

325 330 335
Ile Gly Val Gln Leu Asp Gly Thr Glu Arg Leu His Gly Ala Ala Glu
                340                 345                 350
Arg Glu Ala Val Met Leu Ile Lys Lys Thr Ala Phe Gln Ile Ala Glu
            355                 360                 365
Ala Lys Glu Leu Pro Met Ser Cys Ala Gln Glu Ser Ile Thr Ser Leu
        370                 375                 380
Tyr Lys Lys Ala Gly Ser Glu Asn Leu Tyr Phe Gln
    385                 390                 395

## Beschreibung

Die Erfindung betrifft das allgemeine Gebiet der Optogenetik.

Es wird ein Verfahren zur Optimierung und Veränderung des Metaboloms durch optogenetische Kontrolle des Zellzyklus von Organismen wie z.B. *Saccharomyces cerevisiae* und anderen Eukaryoten bereitgestellt zur Steigerung der Produktion von kommerziell interessanten Stoffwechselprodukten wie z.B. den Pflanzen- oder Pilzinhaltsstoffen β-Carotin, Gibberellin oder Cordycepin.

Mit diesem Verfahren wird die Ausbeute an β-Carotin gegenüber herkömmlichen Verfahren um 100% gesteigert. Das Verfahren ist auch für die Herstellung von anderen kommerziell interessanten Stoffwechselprodukten für Proteine, Nukleinsäuren oder Lipide geeignet.

### Stand der Technik

Die Optogenetik ist eine sehr junge biologische Technologie, die erst in den letzten 10 Jahren bekannt wurde. Sie wird angewendet, um zelluläre Aktivität in einem Organismus mit Licht kontrollieren zu können. Dabei verknüpft sie genetische und optische Methoden, so dass bestimmte Ereignisse und Vorgänge in spezifischen Zellen lebender Organismen mit Licht ausgelöst oder reguliert werden.

Dazu werden spezifisch Fremdgene in die Zielzellen eingeschleust, die zur Expression lichtempfindlicher lonenkanäle, Transporter, Transkriptionsfaktoren oder Enzyme führen.

Diese spezifischen Fremdgene kodieren für Opsin oder Opsin-Mutanten, z.B. für Channelrhodopsin-2, auch nichtselektiver Kationenkanal oder kurz ChR-2 genannt. Alternativ werden Gene von Halorhodopsin (NpHR), einer Chloridpumpe oder die Guanylyl-Cyclase aus dem Pilz *Blastocladiella emersonii* verwendet, da sie sehr effizient und zuverlässig auf Licht reagieren. An Mäusen konnte gezeigt werden, wie dieser Lichteinfluss auf lebende Zellen wirkt. Wird der nichtselektive Kationenkanal ChR-2 in Neuronen exprimiert, so führt der Einfall von blauem Licht zu einer Depolarisation also einer Aktivierung der Neuronen in den Mäusen. Wird NpHR in Neuronen exprimiert, so führt der Einfall von gelbem Licht zu einer Hyperpolarisation also einer Inaktivierung der Neuronen in den Mäusen. Neurone können in lebenden Organismen also auf diese Art an- und abgeschaltet werden. Gezeigt wurde auch die lichtgesteuerte Bewegungskontrolle von *Caenorhabditis elegans.*

Die spezifischen Fremdgene kommen nicht natürlicherweise in den Organismen vor, die mit Lichteinfluss reguliert werden sollen. Sie werden eingeschleust z.B. über Transduktion mit Viren oder aber es werden transgene Organismen erzeugt, die die Fremdgene dann schon im gesamten Erbgut tragen.

Das Licht wird z.B. durch feine Glasfaserkabel, an denen Lichtquellen wie z.B. LEDs gekoppelt sind, an die Zelle herangeführt, wie im Fall der Neuronen in lebenden Mäusen.

Es ist auch möglich die Expression von Genen durch lichtgesteuerte Promotoren zu regulieren. Bekannt sind Anwendungen der Optogenetik zu medizinisch und therapeutisch relevanten Fragestellungen. Der Fokus liegt dabei auf den Erkrankungen Makuladegeneration, Parkinson, Schlaganfall und Epilepsie.

Der Pflanzeninhaltsstoff β-Carotin ist ein kommerziell sehr interessantes Stoffwechselprodukt. β-Carotin ist ein Wirkstoff aus der Gruppe der Carotinoide und ein natürlicher Farb- und Inhaltsstoff von vielen Früchten und Gemüsen, beispielsweise Karotten, Tomaten, Spinat, Mangos und Aprikosen. β-Carotin hat antioxidative Eigenschaften und kann insbesondere im Darm zu Vitamin A umgewandelt werden. Die herkömmlichen Verfahren zur Steigerung der Expression von β-Carotin in der Bäckerhefe, *Saccharomyces cerevisiae* umfassen einerseits die Verbesserung der Promotoren oder eine Optimierung des Mediums durch Zugabe von Acetat.

Der Pflanzeninhaltsstoff Gibberellin ist ebenfalls kommerziell sehr interessant. Gibberelline, auch GA abgekürzt, sind eine Gruppe von Pflanzenhormonen, die u.a. die Samenkeimung fördern, das Streckungswachstum der Sprossachse kontrollieren sowie außerdem die Blühinduktion beeinflussen. Gibberelline zählen chemisch zu den Terpenoiden und leiten sich somit vom Isopren ab (Isoprenoide). Inzwischen sind über 100 verschiedene Gibberelline beschrieben worden. Sie alle zeichnen sich durch das Vorhandensein eines *ent*-Kauren-Ringes aus, der sich auch im *ent-*Gibberellan-Grundgerüst wiederfindet, auf dem alle Gibberelline basieren. Alle bislang bekannten Gibberelline werden der Einfachheit halber von GA1 bis GA125 durchnummeriert. Gibberellin hat eine große Bedeutung bei der Fruchtproduktion, indem sie z.B. das Stängelwachstum bei kernlosen Trauben so fördern, dass die Beeren nicht zu eng wachsen und ihre erwünschte Größe erreichen. In ähnlicher Weise werden Gibberelline auch beim Bierbrauen eingesetzt, um die Malzbildung zu beschleunigen. GA1 und GA4 sind bisher nur schwer synthetisch zugänglich, jedoch ist vor allem GA4 von besonderem Interesse, da es zum Beispiel in Reispflanzen das wichtigste Gibberellin mit der höchsten biologischen Aktivität darstellt. Es besteht ein erhöhter Bedarf an kommerziell interessanten Stoffwechselprodukten aus Organismen. Dazu ist die Optimierung und Veränderung des Metaboloms dieser Organismen, die diese Stoffwechselprodukte herstellen können, wünschenswert. Dem Fachmann ist bekannt, dass sich das Metabolom beim Durchlaufen des Zellzyklus verändert.

Bisher wurden Veränderungen und Optimierungen des Metaboloms durch dauerhafte Veränderung des Genoms realisiert z.B. Deletionen oder durch temporäre Kontrolle konkurrierenden zellulärer Prozesse über die Temperatur oder durch Zugabe von Stoffen als Inhibitoren oder anderen Induktoren in die Zellkultur. Die bisherigen auf Genveränderung basierenden Verfahren bringen diverse Nachteile mit sich, weil eine dauerhafte Veränderung keine Regulierung mehr ermöglicht oder das Zellwachstum erheblich eingeschränkt wird. Die bisherigen auf Temperaturkontrolle basierenden Verfahren sind teuer, benötigen Zeit und sind teilweise inkompatibel mit bestimmten Organismen, die ein anderes Temperaturoptimum haben. Die bisherigen auf Zugabe von Stoffen als Inhibitoren oder anderen Induktoren basierenden Verfahren sind ebenfalls teuer, schwer kontrollierbar und erzeugen manchmal störende Abbauprodukte durch die Metabolisierung der zusätzlichen Stoffe.

### Aufgabe

Aufgabe der vorliegenden Erfindung ist es die Nachteile im Stand der Technik zu beseitigen und ein verbessertes Verfahren zur Herstellung von ausgewählten, pflanzlichen oder pilzlichen Inhaltsstoffen oder anderen biosynthetisch zugänglichen Wirkstoffen bereitzustellen.

### Lösung der Aufgabe

Diese Aufgabe wird erfindungsgemäß gelöst durch die Merkmale des Anspruch 1. Weitere vorteilhafte Ausführungsformen sind den Unteransprüchen zu entnehmen.

Insbesondere handelt es sich um ein Verfahren zur Herstellung eines pflanzlichen oder pilzlichen Inhaltsstoffes in eukaryotischen Zellen durch optogenetische Kontrolle von mindestens einem Zellzyklusregulator umfassend die Schritte
i) Bereitstellen eines Expressionsvektors, der ein photosensitives Degron aufweist, welches bei Blaulichtexposition zwischen 420-490 nm eine Degradationssequenz freisetzt, welche durch das Ubiquitin-Proteasomen System erkannt wird und wobei der Expressionsvektor für das Protein mindestens eines Zellzyklusregulators kodiert sodass ein Fusionsprotein mit dem photosensitiven Degron entsteht.
ii) Transfektion des Expressionsvektors in eukaryotische Zellen, die den pflanzlichen oder pilzlichen Inhaltsstoff exprimieren, um dessen Produktion durch Blaulichtexposition zwischen 420-490 nm steuerbar zu machen.
iii) Exposition der eukaryotischen Zellen bei Blaulicht zwischen 420-490 nm so dass das Fusionsprotein abgebaut und der pflanzliche oder pilzliche Inhaltsstoff in den eukaryotischen Zellen gesteigert freigesetzt wird.

Dabei ist der mindestens eine Zellzyklusregulator ausgewählt aus der Gruppe Cdc48, Npl4, Sic1 und Clb2. Dabei sind die eukaryotischen Zellen Hefen. Der Pflanzeninhaltsstoff ist β-Carotin oder Gibberellin, der pilzliche Stoff ist Cordycepin. Die Steigerung umfasst mindestens 100% gegenüber einem Wildtyp. Das photosensitive Degron umfasst eine Photorezeptordomäne, wobei eine lichtabhängige strukturelle Änderung eine Degradationssequenz freisetzt zur Erkennung durch das Ubiquitin-Proteasomensystem. Das photosensitive Degron umfasst eine Photorezeptordomäne eines pflanzlichen Phototropins aus der Light Oxygen Voltage (LOV) Familie. Die Degradationssequenz umfasst die C-terminale Degradationssequenz einer tierischen Ornithindecarboxylase.

Ein neuer Ansatz ist es, die gewünschte Optimierung und Veränderung des Metaboloms von Organismen wie z.B. *Saccharomyces cerevisiae* und anderen Eukaryoten durch optogenetische Kontrolle des Zellzyklus zu realisieren, um die Expression und Synthese von kommerziell interessanten Stoffwechselprodukten zu steigern.

Mit dem vorliegenden Verfahren wird die Transkription exemplarisch in einem Pilz, beispielhaft die Bäckerhefe (*Saccharomyces cerevisiae*) optogenetisch kontrolliert, wobei die optogenetische Kontrolle den Licht-induzierten Abbau von Zellzyklusregulatoren oder die Licht-gesteuerte Biosynthese von heterologen Enzymen umfasst.

Das erfindungsgemäße Verfahren zur Herstellung eines Pflanzeninhaltsstoffes durch optogenetische Kontrolle von Zellzyklusregulatoren basiert auf dem Bereitstellen eines Expressionsvektors, der ein photosensitives Degron aufweist, dass bei Blaulichtexposition zwischen 420-490 nm eine Degradationssequenz freisetzt, welche durch das Ubiquitin-Proteasomen System erkannt wird, sowie für das Protein eines Zellzyklusregulators kodiert. Mit diesem photosensitiven Degron können dominant-negative Zellzyklusregulatoren wie Clb2^{ΔDB} oder ^{ΔN}Sic1 modifiziert werden oder das kodierende Gen kann chromosomal an Gene fusioniert werden, die für Zellzyklusregulatoren kodieren. Blaulichtexposition zwischen 420-490 nm induziert den Abbau des Fusionsproteins aus Zellzyklusregulator und photosensitivem Degron. Im Falle von Clb2^{ΔDB}-psd³ oder ^{ΔN}Sic1-psd³ führt also Dunkelheit zu einem Zellzyklusarrest (Akkumulation der dominant-negativen Regulatoren) im Falle anderer Zellzyklusregulatoren führt das Fehlen der Regulatoren nach Blaulichtinkubation zu einem Arrest. Das erfindungsgemäße Verfahren zeigt bei gleichzeitiger Expression eines heterologen Biosyntheseweges in diesen Zellen (z.B. für β-Carotin) Ausbeutesteigerung für die Biosynthese des Zielproduktes. Bevorzugt werden dabei die Gene der Zellzyklusregulatoren Cdc48-psd³ und Npl4-psd¹ bzw. ^{ΔN}SiC1-psd³ und Clb2^{ΔDB}-psd³ verwendet, die eine effiziente Regulation des Zellzyklus durch Licht ermöglichen. Der Zellzyklus ist die Abfolge verschiedener Aktivitätsphasen zwischen den Teilungen eukaryotischer Zellen. Da der DNA-Gehalt einer Zelle bzw. eines Zellkerns bei der Teilung (Mitose) halbiert wird, muss er vor der nächsten Teilung wieder verdoppelt werden.

Diese beiden Vorgänge werden als *M-Phase* und *S-Phase* (von Synthese) bezeichnet. Zwischen ihnen liegen sogenannte Gap-Phasen (engl. *Lücke*): *G₁* und *G*₂. Aus einer Mutterzelle werden zwei identische Tochterzellen.

Zellzyklusregulatoren sind Proteine, die regulativ am Zellzyklus beteiligt sind. Dies sind z.B. die Cycline und die CDKs (*Cyclin Dependent Kinases*). Zu bestimmten Zeitpunkten im Zyklus werden diese Proteine verstärkt exprimiert, bis ihre Konzentration ein Maximum erreicht. Von diesem Maximum nimmt man an, dass es den Kontrollpunkt darstellt. Danach werden die Cycline schnell abgebaut. CDKs und die zugehörigen Cycline bilden Komplexe, deren Aktivierung (Dephosphorylierung von Thr14 und Tyr15 durch cdc25) beziehungsweise Deaktivierung unter anderem durch Wachstumsfaktoren und Protoonkogene gesteuert wird. Die CDKs phosphorylieren und aktivieren spezifisch eine Reihe anderer Proteine und steuern so den Zellzyklus. Der Zellzyklusregulator Sic1 ist ein Inhibitor von Cdk1 und verhindert die DNA-Synthese in der S-Phase. Sic1 wird durch Proteolyse entfernt, wodurch Cdk1 aktiviert wird. Das Cyclin Clb2 aktiviert Cdk1 und wird vor Eintritt in die Mitose abgebaut. Wird der Abbau verhindert, verharrt die Zelle in der G2-Phase. Cdc48 und Npl4 sind indirekte Zellzyklusregulatoren, da sie Teil der Abbaumaschinerie sind, die zur Entfernung von Zellzyklusregulatoren wie Clb2 benötigt werden. Daher kann man bei Entfernung von Cdc48 oder Npl4 oder anderer am Abbau von Zellzyklusregulatoren beteiligter Proteine eine Arretierung in einer Phase des Zellzyklus erreichen.

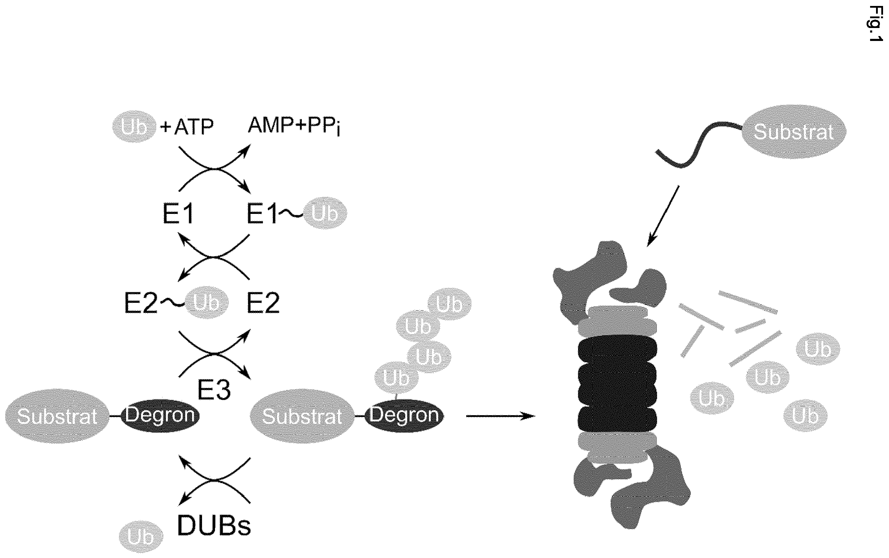

Die Licht-abhängige Regulation der Zellzyklusregulatoren kann über verschiedene Wege erreicht werden. Gemeinsam ist jeweils eine Degradationssequenz (Degron), die durch Exposition der Zellen mit Licht aktiviert wird. Das photosensitive Degron (psd) ist der Prototyp einer Licht-aktivierbaren Degradationssequenz. Es besteht aus einem Photorezeptor der Light Oxygen Voltage (LOV) Familie. Die benutzte Variante stammt von der 2. LOV Domäne des *Avena sativa* Proteins Phototropin ab und wurde durch Zufallsmutagenese und Selektion erhalten. Die zweite Komponente ist eine synthetische Degradationssequenz die von dem Mausprotein Ornithindecarboxylase abstammt. Der Photorezeptor präsentiert nach Blaulichtexposition (= Licht mit Wellenlänge zwischen 420-490 nm) die Degradationssequenz, diese wird dann vom Ubiquitin-Proteasomensystem erkannt und induziert dadurch den Abbau des Fusionsproteins. Das Ubiquitin-Proteasomensystem ist in eukaryotischen Zellen die Hauptmaschinerie für die geregelte Proteolyse von Proteinen, die im Zytosol oder dem Zellkern lokalisiert sind. Das Proteasom ist ein selbst-kompartimentierender Proteasekomplex. Proteine werden durch spezielle Enzyme zum Abbau markiert und anschließend im Proteasom in Peptide zerlegt, wie Figur 1 zeigt. Durch Fusion des photosensitiven Degrons an Proteine wie Cdc48 oder Npl4 kann ihre Degradation durch Licht induziert werden. Dies geschieht typischerweise durch chromosomale Integration des Gens welches für das photosensitive Degron Modul kodiert an das 3'-Ende der für Cdc48 oder Npl4 kodierenden Gene. Die Vorgehensweise ist dem Fachmann bekannt. Dadurch liegt jedes Cdc48 oder Npl4 Molekül als Fusionsprotein (Cdc48-psd³ bzw. Npl4-psd¹) vor. Lichtexposition der Zellen führt zum Abbau von Cdc48-psd³ oder Npl4-psd¹ und zur Arretierung der Zellen in der G2-Phase des Zellzyklus durch Akkumulation von nichtabgebauten Zellzyklusregulatoren.

Eine weitere mögliche Variante besteht darin, eine Genfusion herzustellen, bestehend aus einer Dominant-negativen Variante eines Zellzyklusregulators wie Clb2^{ΔDB} oder ^{ΔN}Sic1 und dem photosensitiven Degron. Um die dominant-negativen Varianten zu erzeugen, wurden regulatorische Sequenzen aus Sic1 und Clb2 entfernt, die normalerweise die Zellzyklus-abhängige Proteolyse dieser Proteine steuern. Das psd³ Modul ersetzt somit die Zellzyklus-abhängige Proteolyse der Regulatoren durch Blaulicht-gesteuerte Degradation. Diese Varianten werden zusätzlich zu den nativen Genen für Clb2 und Sic1 exprimiert und liegen Plasmid-kodiert vor. Dadurch ist keine aufwändige genetische Manipulation der chromosomalen Gene notwendig. In Hefezellen, die in Dunkelheit inkubiert werden, arretieren die Zellen im Fall von Clb2^{ΔDB}-psd³ in der G2-Phase des Zellzyklus, im Fall von ^{ΔN}Sic1-psd³ in der G1-Phase.

Die geschilderte Vorgehensweise ist analog auch in anderen Eukaryoten möglich, es sind beispielsweise ähnliche Licht-aktivierbare Degronkonstrukte für Säugerzellkultur oder Zebrafischzellen bekannt und das psd-Modul funktioniert auch im Fadenwurm.

Es wurden verschiedene photoaktivierbare Moleküle entwickelt, die in Bäckerhefe, Säugerzellen und Zebrafischen zur Licht-induzierten Degradation verwendet wurden. Allerdings sind die verwendeten Moleküle SpezialChemikalien, daher ist der Einsatz in der Biotechnologie nicht kostengünstig möglich.

Der heterologe Syntheseweg für β-Carotin wird im Ausführungsbeispiel ständig in Hefe exprimiert, die Arretierung im Zellzyklus führt zu einer Erhöhung der Produktausbeute. Beispielhaft werden der Pflanzeninhaltsstoff β-Carotin, Gibberellin oder der pilzliche Stoff Cordycepin hergestellt. Beispielhaft erfolgt die Synthese in der Bäckerhefe *Saccharomyces cerevisiae.* Dies ist aber nur exemplarisch zu verstehen, generell kann die Synthese auch in allen anderen Eukaryoten stattfinden. Interessant für die Anwendung sind vor allem eukaryotische Mikroorganismen (z.B. einzellige Pilze oder tierische Zelllinien), die unter Kultivierungsbedingungen leicht mit blauem Licht bestrahlt werden können.

Messungen des zellulären DNA-, RNA-, Protein-, Kohlenhydrat- und Lipidgehalts zeigen deutliche Veränderungen nach Arretierung des Zellzyklus. Kürzlich publizierte Studien belegen die Zellzyklusabhängigkeit des Metabolismus. Das vorgestellte Verfahren erlaubt die gezielte Arretierung der Zellen in einer bestimmten Zellzyklusphase. Dies kann zur Ausbeutesteigerung in biotechnologischen Prozessen ausgenutzt werden.

Das erfindungsgemäße Verfahren ermöglicht die gezielte Steuerung bzw. Synchronisation der Zellzyklus-Regulatoren, die zu einer unerwarteten Steigerung der Ausbeute an Stoffwechselprodukten führen. Somit können kommerziell relevante Mengen von Stoffwechselprodukten wie z.B. Pflanzenwachstumshormone, wie Gibberellin und β-Carotin auf kostengünstigem Weg hergestellt werden. Ein Ausführungsbeispiel zeigt die erfindungsgemäße Produktion von Cordycepin, einem Derivat des Nucleosids Adenosin, das sich von letzterem durch das Fehlen der Hydroxygruppe in der 3'-Position seiner Riboseeinheit unterscheidet. Es stammt aus dem Pilz *Cordyceps militaris.*

### Ausführungsbeispiel β-Carotinsynthese

Seq.ID 1 Aminosäuresequenz Licht-regulierbarer Zellzyklusregulator
Cdc48-psd¹:
MGEEHKPLLDASGVDPREEDKTATAILRRKKKDNMLLVDDAINDDNSVIAINSNT
MDKLELFRGDTVLVKGKKRKDTVLIVLIDDELEDGACRINRVVRNNLRIRLGDLVTI
HPCPDIKYATRISVLPIADTIEGITGNLFDVFLKPYFVEAYRPVRKGDHFVVRGGM
RQVEFKVVDVEPEEYAVVAQDTIIHWEGEPINREDEENNMNEVGYDDIGGCRKQ
MAQIREMVELPLRHPQLFKAIGIKPPRGVLMYGPPGTGKTLMARAVANETGAFFF
LINGPEVMSKMAGESESNLRKAFEEAEKNAPAIIFIDEIDSIAPKRDKTNGEVERRV
VSQLLTLMDGMKARSNVVVIAATNRPNSIDPALRRFGRFDREVDIGIPDATGRLE
VLRIHTKNMKLADDVDLEALAAETHGYVGADIASLCSEAAMQQIREKMDLIDLDED
EIDAEVLDSLGVTMDNFRFALGNSNPSALRETVVESVNVTWDDVGGLDEIKEELK
ETVEYPVLHPDQYTKFGLSPSKGVLFYGPPGTGKTLLAKAVATEVSANFISVKGP
ELLSMWYGESESNIRDIFDKARAAAPTVVFLDELDSIAKARGGSLGDAGGASDRV
VNQLLTEMDGMNAKKNVFVIGATNRPDQIDPAILRPGRLDQLIYVPLPDENARLSI
LNAQLRKTPLEPGLELTAIAKATQGFSGADLLYIVQRAAKYAIKDSIEAHRQHEAE
KEVKVEGEDVEMTDEGAKAEQEPEVDPVPYITKEHFAEAMKTAKRSVSDAELRR
YEAYSQQMKASRGQFSNFNFNDAPLGTTATDNANSNNSAPSGAGAAFGSNAEE
DDDLYSRTLQVDGSSRGEQKLISEEDLNGEQKLISEEDLNGEQKLISEEDLNGSS
RVMRKGIDLATTLERIEKNFVITDPRLPDNPIIFASDSFLELTEYSREEILGRNCRFL
QGPETDLTTVKKIRNAIDNQTEVTVQLINYTKSGKKFWNIFHLQPMRDQKGEVQY
FIGVQLDGSKHVEPVRNVIEETAVKEGEDLVKKTAVNIDEAVRELPMSCAQESITS
LYKKAGSENLYFQ

Seq.ID 2 Aminosäuresequenz Licht-regulierbarer Zellzyklusregulator
Cdc48-psd³:
MGEEHKPLLDASGVDPREEDKTATAILRRKKKDNMLLVDDAINDDNSVIAINSNT
MDKLELFRGDTVLVKGKKRKDTVLIVLIDDELEDGACRINRVVRNNLRIRLGDLVTI
HPCPDIKYATRISVLPIADTIEGITGNLFDVFLKPYFVEAYRPVRKGDHFVVRGGM
RQVEFKVVDVEPEEYAVVAQDTIIHWEGEPINREDEENNMNEVGYDDIGGCRKQ
MAQIREMVELPLRHPQLFKAIGIKPPRGVLMYGPPGTGKTLMARAVANETGAFFF
LINGPEVMSKMAGESESNLRKAFEEAEKNAPAIIFIDEIDSIAPKRDKTNGEVERRV
VSQLLTLMDGMKARSNVVVIAATNRPNSIDPALRRFGRFDREVDIGIPDATGRLEV
LRIHTKNMKLADDVDLEALAAETHGYVGADIASLCSEAAMQQIREKMDLIDLDEDE
IDAEVLDSLGVTMDNFRFALGNSNPSALRETVVESVNVTWDDVGGLDEIKEELKE
TVEYPVLHPDQYTKFGLSPSKGVLFYGPPGTGKTLLAKAVATEVSANFISVKGPE
LLSMWYGESESNIRDIFDKARAAAPTVVFLDELDSIAKARGGSLGDAGGASDRVV
NQLLTEMDGMNAKKNVFVIGATNRPDQIDPAILRPGRLDQLIYVPLPDENARLSIL
NAQLRKTPLEPGLELTAIAKATQGFSGADLLYIVQRAAKYAIKDSIEAHRQHEAEK
EVKVEGEDVEMTDEGAKAEQEPEVDPVPYITKEHFAEAMKTAKRSVSDAELRRY
EAYSQQMKASRGQFSNFNFNDAPLGTTATDNANSNNSAPSGAGAAFGSNAEED
DDLYSRTLQVDGSSRGEQKLISEEDLNGEQKLISEEDLNGEQKLISEEDLNGSSR
VMRRGIDLATTLERIEKNFVITDPRLPDNPIIFASDSFLQLTEYSREEILGRNCRFLQ
GPETDRATVRKIRDAIDNQTEVTVQLINYTKSGKKFWNVFHLQPMRDYKGDVQY
FIGVQLDGTERLHGAAEREAVMLIKKTAFQIAEAKELPMSCAQESITSLYKKAGSE
NLYFQ

Seq.ID 3 Aminosäuresequenz Licht-regulierbarer Zellzyklusregulator
Npl4-psd¹:
MLIRFRSKNGTHRVSCQENDLFGTVIEKLVGNLDPNADVDTFTVCEKPGQGIHAV
SELADRTVMDLGLKHGDMLILNYSDKPANEKDGVNVEIGSVGIDSKGIRQHRYGP
LRIKELAVDEELEKEDGLIPRQKSKLCKHGDRGMCEYCSPLPPWDKEYHEKNKIK
HISFHSYLKKLNENANKKENGSSYISPLSEPDFRINKRCHNGHEPWPRGICSKCQ
PSAITLQQQEFRMVDHVEFQKSEIINEFIQAWRYTGMQRFGYMYGSYSKYDNTPL
GIKAVVEAIYEPPQHDEQDGLTMDVEQVKNEMLQIDRQAQEMGLSRIGLIFTDLS
DAGAGDGSVFCKRHKDSFFLSSLEVIMAARHQTRHPNVSKYSEQGFFSSKFVTC
VISGNLEGEIDISSYQVSTEAEALVTADMISGSTFPSMAYINDTTDERYVPEIFYMK
SNEYGITVKENAKPAFPVDYLLVTLTHGFPNTDTETNSKFVSSTGFPWSNRQAM
GQSQDYQELKKYLFNVASSGDFNLLHEKISNFHLLLYINSLQILSPDEWKLLIESAV
KNEWEESLLKLVSSAGWQTLVMILQESGRTLQVDGSSRGEQKLISEEDLNGEQK
LISEEDLNGEQKLISEEDLNGSSRVMRKGIDLATTLERIEKNFVITDPRLPDNPIIFA
SDSFLELTEYSREEILGRNCRFLQGPETDLTTVKKIRNAIDNQTEVTVQLINYTKSG
KKFWNIFHLQPMRDQKGEVQYFIGVQLDGSKHVEPVRNVIEETAVKEGEDLVKK
TAVNIDEAVRELPMSCAQESITSLYKKAGSENLYFQ

Seq.ID 4 Aminosäuresequenz Licht-regulierbarer Zellzyklusregulator
Clb2^ΔDB-psd^3:

MSNPIENTENSQNTSSSRFLRNVQTTFQKSNANNPALTNFKSTLNSVKKE
GSRIPQFTRESVSRSTAAQEEKRTLKENGIQLPKNNLLDDKENQDPSSQQ
FGALTSIKEGRAELPANISLQESSSAKEIIQHDPLKGVGSSTEVVHNSVENE
KLHPARSQLQVRNTESETDSGKKRPISTIVEQELPKKFKVCDENGKEEYE
WEDLDAEDVNDPFMVSEYVNDIFEYLHQLEVITLPKKEDLYQHRNIHQNR
DILVNWLVKIHNKFGLLPETLYLAINIMDRFLGKELVQLDKLQLVGTSCLFIA
SKYEEVYSPSIKHFASETDGACTEDEIKEGEKFILKTLKFNLNYPNPMNFLR
RISKADDYDIQSRTLAKFLLEISLVDFRFIGILPSLCAAAAMFMSRKMLGKG
KWDGNLIHYSGGYTKEELAPVCHMIMDYLVSPIVHDEFHRKYQSRRFMKA
SIISVQWALKVRKNGYDIMTLHESRGEQKLISEEDLNGEQKLISEEDLNGE
QKLISEEDLNGSSRVMRRGIDLATTLERIEKNFVITDPRLPDNPIIFASDSFL
QLTEYSREEILGRNCRFLQGPETDRATVRKIRDAIDNQTEVTVQLINYTKS
GKKFWNVFHLQPMRDYKGDVQYFIGVQLDGTERLHGAAEREAVMLIKKT
AFQIAEAKELPMSCAQESITSLYKKAGSENLYFQ

Seq.ID 5 Aminosäuresequenz Licht-regulierbarer Zellzyklusregulator
ΔNSic1-psd3:

MEQEPLGRVNRILFPTQQNVDIDAAEEEEEGEVLLPPSRPTSARQLHLSLERDEF
DQTHRKKIIKDVPGTPSDKVITFELAKNWNNNSPKNDARSQESEDEEDIIINPVRV
GKNPFASDELVTQEIRNERKRAMLRENPDIEDVITYVNKKGEVVEKRRLTDEEKR
RFKPKALFQSRDQEHSRGEQKLISEEDLNGEQKLISEEDLNGEQKLISEEDLNGS
SRVMRRGIDLATTLERIEKNFVITDPRLPDNPIIFASDSFLQLTEYSREEILGRNCRF
LQGPETDRATVRKIRDAIDNQTEVTVQLINYTKSGKKFWNVFHLQPMRDYKGDV
QYFIGVQLDGTERLHGAAEREAVMLIKKTAFQIAEAKELPMSCAQESITSLYKKAG
SENLYFQ

Der β-Carotinsyntheseweg wird durch Transformation mit dem Plasmid pUDE269¹⁶ in Hefezellen implementiert. Die Transformation von Mikroorganismen, wie z.B. Hefe, ist dem Fachmann bekannt. Zur Kultivierung der Hefezellen wird ein geeignetes Kulturmedium verwendet z.B. ein modifiziertes WM8 Medium.

Modifiziertes WM8 Medium wird wie folgt angesetzt:
Vorbereitend wird eine 10fach konzentrierte Salzlösung (10 g KH₂PO₄; 2,5 g MgCl₂ 6H₂O; 1 g NaCl; 1 g CaCl₂; 28 g NH₄Cl in 1 l demineralisiertem Wasser), eine 100fach konzentrierte Spurenelementlösung (10 mg H₃BO₃; 4 mg CuSO₄▪5H₂O; 3 mg CoCl₂ 6H₂O; 1 mg Kl; 35 mg FeSO₄ 7H₂O; 20 mg MnCl2 4H₂O; 5 mg Na₂MoO₄ 2H₂O; 62,5 mg ZnSO₄ 7H₂O in 100ml demineralisiertem Wasser) und eine 100fach konzentrierte Vitaminlösung (25,5 mg Biotin; 510 mg Calciumpantothenat; 1g *myo-*Inositol; 110 mg Nicotinsäure; 2 mg p-Aminobenzoesäure; 260 mg Pyridoxin-HCl; 110 mg Thiamin-HCI in 100 ml demineralisiertem Wasser) angesetzt. Für einen Liter WM8+ Medium werden 100 ml Salzlösung, 10 ml Spurenelementlösung, 10 ml Vitaminlösung, 50 g Glukose, 10 g Natriumglutamat und 2 g Aminosäurenmix umfassend 5 g Adenin; 20 g Alanin; 20 g Arginin; 20 g Asparagin; 20 g Asparaginsäure; 20 g Cystein; 20 g Glutamin; 20 g Glutaminsäure; 20 g Glycin; 20 g *myo-*Inositol; 20 g Isoleucin; 20 g Lysin; 20 g Methionin; 2 g para-Aminobenzoesäure; 20 g Phenylalanin; 20 g Prolin; 20 g Serin; 20 g Threonin; 20 g Tyrosin; 20 g Valin gut durchmischt. Zu 36,7 g des Gemischs werden gegebenenfalls zum Ausgleich eventuell vorhandener Auxotrophien Histidin (2 g), Leucin (4 g), Uracil (2 g) oder Tryptophan (2 g) hinzugefügt) vermischt und mit demineralisiertem Wasser zu einem Liter Endvolumen aufgefüllt. Das Medium wird anschließend durch 10-minütiges Autoklavieren sterilisiert.

Es werden Vorkulturen der Mutanten-Stämme (Cdc48-psd^{1/3}, Npl4-psd¹) und isogenen Wildtyp-Stämme hergestellt. Dazu werden z.B. einzelne Kolonien in 3 ml WM8 (-URA) in Reagenzgläsern angesetzt und über Nacht schüttelnd bei 30°C inkubiert. Aus dieser Vorkultur wird anschließend eine größere Kultur hergestellt. Dies erfolgt z.B. indem 10 ml Kultur (WM8+-Ura in Plastikflaschen für Zellkultur) in einem Verhältnis 1:50 beimpft wird und für 24 h bei 30°C im Dunkeln schüttelnd (90 UPM) inkubiert wird.

Durchführung von mindestens einer Beleuchtungsphase, so dass lichtinduziert ein Zellzyklus-Stopp und eine Arretierung des Wachstums erfolgt:
Nach der Wachstumsphase werden die Kulturen in geeignete Behälter z.B. Boxen überführt. Diese Boxen sind vorzugsweise mit Spiegelfolie ausgekleidet und tragen RGB-LEDs zur Beleuchtung der Zellen im Deckel. Die Kulturen werden inkubiert, z.B. für 24 h bei 30°C und 90 UPM geschüttelt. Während dieser Zeit werden drei sich wiederholende Beleuchtungszyklen durchlaufen, ein Zyklus bestehend aus 5 h Blaulicht-Beleuchtung (470nm), gefolgt von 3h Dunkelheit. Die Lichtintensität beträgt 30 µmol/m²s. Die Lichtphasen bewirken einen psd-bedingten Abbau von Cdc48-psd^{1/3} bzw. Npl4-psd¹, dies führt zu einem Zellzyklus-Stopp und einer Arretierung des Wachstums. Hierdurch steht mehr Energie für die heterologe Produktion von β-Carotin zur Verfügung.

Durchführung von mindestens einer Dunkelphase für erhöhte Produktion von β-Carotin:
Die Dunkelphase dauert 3 h, so dass der Abbau von Cdc48-psd^{1/3} unterbrochen und der Zellzyklus fortgesetzt wird. Diese Dunkelphase dient zur Erreichung einer ausreichenden Zellmenge und einer Verringerung des zellulären Stresses.

Vorzugsweise werden drei sich wiederholende Beleuchtungszyklen durchlaufen, wobei ein Zyklus bestehend aus 5h Blaulicht-Beleuchtung (470nm), gefolgt von 3 h Dunkelheit. Die Lichtintensität beträgt 30 µmol/m²s. Die Lichtphasen bewirken einen psd-bedingten Abbau von Cdc48-psd^{1/3} bzw. Npl4-psd¹ mit daraus resultierendem Zellzyklus-Stopp und Arretierung des Wachstums. Hierdurch steht mehr Energie für die heterologe Produktion von β-Carotin zur Verfügung. In den Dunkelphasen wird der Abbau von Cdc48-psd^{1/3} unterbrochen und der Zellzyklus fortgesetzt. Diese Dunkelintervalle dienen zur Erreichung einer ausreichenden Zellmenge und einer Verringerung des zellulären Stresses. Nach der 24 h-Hauptwachstumsphase und der 24 h-Intervallphase werden Proben für die Analyse z.B. im Durchflusszytometer entnommen (30 µl) und die restliche Kulturlösung bei 3000 UPM für 3 min zentrifugiert, der Überstand entfernt und das Zellpellet gefriergetrocknet.

Im Falle der Stämme mit *^{ΔN}SIC1-psd³* und *CLB2^{ΔDB}-psd³* erfolgt die Kultivierung analog zu *CDC48-psd³* mit der Ausnahme, dass hier die Zellen konstant 12 h lang bei 30 µmol/m²s Blaulicht (420-490 nm) inkubiert werden und zur Arretierung des Zellzyklus für 24 h in Dunkelheit überführt werden. Als Medium wird Minimalmedium (WM8+ -Ura-Leu) verwendet. Zur Analyse im Durchflusszytometer werden die Zellkulturen 1:200 mit Wasser verdünnt und Stoffwechsel-Prozesse mittels Kühlung auf Eis und Zugabe von Natriumazid (Endkonzentration 10 mM) gestoppt. Die Analyse der Fluoreszenz der Zellen erfolgte bei 530nm, die Anregung der Fluoreszenz bei 470 nm. Durch parallele Messung analoger Stämme ohne Plasmid zur β-CarotinSynthese (pUDE269) als Kontrolle wird die Hintergrundfluoreszenz ermittelt, die bei der Berechnung der endgültigen Fluoreszenzintensität notwendig ist. Diese dient zur Ableitung der Menge an produziertem β-Carotin. Anschließend wird die gemessene Fluoreszenz relativ zu einem unmodifizierten Stamm mit pUDE269 bestimmt, um etwaige Verbesserungen der Produktionsmenge festzustellen.

Für die Extraktion des β-Carotins aus den Zellen werden diese in ein 2 ml Reaktionsgefäß überführt und mit 200 µl Glasperlen (Ø 0,4 mm) und 1 ml Extraktionsmedium (50% v/v Methanol, 50% v/v Acetonitril) gemischt und in einem Homogenizer, z.B. dem FastPrep-24 Tissue and Cell Homogenizer bei 6,5 m/s für 40 s aufgeschlossen. Anschließend werden die Zelltrümmer bei 13000 UPM für 5 min abzentrifugiert und der orangegelbe Überstand in ein neues Gefäß überführt. Dieser Schritt wird so lange wiederholt, bis der Überstand und das Zellpellet keine Färbung mehr aufweist. Die so erhaltenen Extrakte werden anschließend per HPLC analysiert. Hierfür wird z.B. ein Agilent 1260 HPLC-System, bestehend aus Entgaser, binärer Pumpe, Säulenofen und MWD-Detektor eingesetzt und die Trennsäule EC 250/4 NUCLEOSIL 100-5 C18 (Macherey-Nagel) verwendet. Die Auftrennung der Probe erfolgt isokratisch mit 50% v/v Methanol, 50% v/v Acetonitril als Laufmittel bei einer Flussrate von 1 ml/min. Das Injektionsvolumen beträgt 25 µl und die Trennsäule wird während der Analyse auf 25°C temperiert. Die Proben werden in Triplikaten vermessen, β-Carotin bei einer Wellenlänge von 450 nm detektiert und mittels Standards quantifiziert. Die erhaltenen Chromatogramme werden mittels Software z.B. der Software OpenLab CDS ChemStation Edition (Agilent Technologies) ausgewertet.

### Ausführungsbeispiel Cordycepinsynthese

Als eine weiteres Ausführungsbeispiel und zur Untermauerung der Messergebnisse bezüglich β-Carotin und der Vielseitigkeit der Zellzyklusarretierung mittels Cdc48-psd³ wird weiterhin ein Stamm mit den Enzymen zur Herstellung von Cordycepin (CNS1/CNS2¹⁷) unter Kontrolle eines blaulichtabhängigen Promoters mit Cdc48-psd³ modifiziert. Dieser wird gleichzeitig mit einem unmodifizierten WT Stamm (+CNS1/CNS2) in geeignetem Minimalmedium (LFM) zunächst genau 12 h in Dunkelheit und anschließend 12 h unter kontinuierlichem Blaulicht bei 30 °C und 90 UPM inkubiert. Durch die Inkubation in Blaulicht wird hierbei sowohl der Zellzyklusarrest durch Degradation von Cdc48-psd³ erreicht als auch mit Hilfe des blaulichtabhängigen Promoters die Cordycepin-Produktion gestartet. Anschließend werden die Zellen abzentrifugiert (4000 UPM, 5 min) und 1 ml des Mediums zur Analyse entnommen. Das Zellpellet wird in 1 ml Wasser aufgenommen und in einem Homogenizer, z.B. dem FastPrep-24 Tissue and Cell Homogenizer bei 6,5 m/s für 40 s aufgeschlossen. Dieses Gemisch wird zusätzlich bei 90°C für 10 min erhitzt und die Zelltrümmer abzentrifugiert (13.000 UPM, 1 min). Das entnommene Kulturmedium wird ebenfalls bei 90°C für 10 min erhitzt. Für die anschließende LC/MS Analyse wird ein Agilent 1100 HPLC-System (Agilent Technologies) gekoppelt mit einem LTQ-FT Ultra Massenspektrometer (Thermo Scientific) verwendet. Das Injektionsvolumen beträgt 20 µl und die Probe wird durch einen Acetonitril/Wasser-Gradienten bei einer Flussrate von 0,2 ml/min über eine Trennsäule z.B. EC 150/2 NU-CLEODUR 100-3 C18ec (Macherey-Nagel) Trennsäule aufgetrennt. Cordycepin wird im ESI-Positivmodus detektiert und über entsprechende Standards quantifiziert. Die erhaltenen Daten werden mit der Software Xcalibur^{™} v2.2 (Thermo Scientific) ausgewertet. Um Wildtyp und Clb2^{ΔDB}-psd³ Zellen für die Biomassebestimmung zu produzieren wachsen zunächst Starterkulturen unter Blaulichtbedingungen in 200 ml LFM (30°C, 100 rpm, 16 h). Mit jeweils 20 ml der Starterkultur werden 10x180 ml LFM innokuliert und die Kulturen für 3 h unter Blaulichtbedingungen geschüttelt (30°C, 100 rpm). Anschließend werden die Kulturen vereinigt und für 24 h in der Dunkelheit geschüttelt (30°C, 100 rpm). Für die Zellernte werden die Kulturen für 20 min bei 1000xg und 4°C zentrifugiert.

Die Zellpellets werden mit 40 ml Wasser gewaschen und in flüssigem Stickstoff gefroren. Abschließend werden die Zellen für 36 h lyophilisiert. Für WT und Clb2^{ΔDB}-psd³ Zellen werden nach diesem Protokoll drei Mal biologische Triplikate produziert. Die Bestimmung des Proteinanteils an der Zelltrockenmasse erfolgt nach Verfahren, die der Fachmann kennt, z.B. über die Biuret-Methode. Hierzu werden lyophilisierte Zellen zu 8 mg/ml in Wasser resuspendiert. Zu 200 µl der Zellsuspension werden 100 µl 1M NaOH gegeben und für 10 min bei 100°C inkubiert. Die Suspension wird anschließend auf Eis gekühlt und nach der Zugabe von 100 µl 0,1M CuSO₄-Lösung 5 min bei RT inkubiert. Die Suspension wird zentrifugiert, 300 µl des Überstandes in eine 96-Well-Mikrotiterplatte überführt und die Absorption bei 550 nm im Mikrotiterplatten Reader ausgelesen. Um die Messung zu kalibrieren, werden Standardlösungen hergestellt aus Rinder-Serumalbumin (BSA) verwendet. Der Kohlenhydrat-Anteil wird über die Phenol-Schwefelsäure-Methode bestimmt. Hierzu werden die getrockneten Zellen zu 0,6 mg/ml in Wasser aufgenommen und 50 µl der Suspension in 96-Well-Mikrotiterplatte pipettiert. Es wird 150 µl 95% H₂SO₄ hinzugegeben und die Suspension gut durchmischt. Anschließend wird 30 µl 5%ige Phenol-Lösung hinzugegeben und die Mikrotiterplatte für 5 min bei 90°C im Wasserbad inkubiert. Schließlich wird die Absorption bei 490 nm mit Mikrotiterplatten Reader ausgelesen. Die Kalibrierung der Messung erfolgt durch Glukose-Standards. Der DNA- und RNA-Gehalt der Proben wird nach Standardverfahren z.B. über die Schmidt-Thannhauser-Schneider Methode bestimmt. Dafür werden zunächst 20 mg Trockenmasse in 1,8 ml eiskaltem 0,25M HClO₄ aufgenommen und für 15 min auf Eis inkubiert. Die Suspension wird zentrifugiert (4.000rpm bei 4°C für 2 min) und der Überstand verworfen. Der Vorgang wird einmal wiederholt, um die Zellen zu waschen. Anschließend wird das Zellpellet in 1,6 ml 0.3M KOH resuspendiert und für 2 h bei 37°C inkubiert. Es werden 200 µl 4,1M HClO₄ hinzugegeben, für 5 min auf Eis inkubiert und zentrifugiert (14.000rpm bei 4°C für 3 min). Zur Bestimmung des RNA-Gehaltes wird der Überstand in ein neues Eppendorf-Gefäß überführt und das Zellpellet für die DNA-Bestimmung zurückgestellt. 360 µl des Überstandes werden mit 250 µl 0,3 M KOH neutralisiert und für 5 min bei Raumtemperatur (17 bis 23 °C) inkubiert.

Die Suspension wird zentrifugiert (14.000 rpm, 2 min) und 15 µl des Überstandes zu 285 µl H₂O in eine UV-transparente 96-Well-Mikrotiterplatten pipettiert. Um den RNA-Gehalt zu bestimmen wird die Absorption der Proben bei einer Wellenlänge von 260nm in einem Mikrotiterplatten Reader bestimmt und die Messung über RNA-Standards kalibriert. Für die DNA-Bestimmung wird das zurückgestellte Zellpellet in 1,8 ml 0,5 M HClO₄ resuspendiert und für 15 min bei 70°C inkubiert. Die Suspension wird zentrifugiert (13.000rpm, 1 min), 800 µl des Überstandes in ein neues Eppendorf-Gefäß überführt und der restliche Überstand verworfen. Anschließend wird das Pellet mit 800 µl eiskaltem 0,25 M HClO₄ gevortext und der Überstand nach Zentrifugieren (13.000rpm, 1 min) verworfen. Die Extraktion wird zweimal wiederholt und die Extrakte vereinigt. Von den vereinigten Extrakten wird 210 µl mit 210 µl Diphenylaminreagenz (1,5 g Diphenylamin, 100 ml HOAc, 1,5 ml H₂SO₄, 0,008% Acetaldehyd) versetzt und 20 h bei 30°C inkubiert. Die Lösung wird zentrifugiert (4.000 rpm, 1 min) und 300 µl in 96-Well-Mikrotiterplatten pipettiert. Abschließend wird die Absorption der Proben bei 595nm im Mikrotiterplatten Reader bestimmt und die Messung mit gleich behandelten DNA-Standards kalibriert. Für die Bestimmung des Lipidgehaltes werden 100 mg Trockenmasse in 1 ml Zymolyasepuffer (50 mM TRIS/HCl pH 7,5, 10 mM EDTA, 0,3 % β-Mercaptoethanol) und Zymolyase (0,05 U/ml) für 1,5 h bei 37°C inkubiert. Die verdauten Zellen werden über Nacht lyophilisiert und das Pellet am nächsten Tag in 200 µl Wasser und 1,25 ml MeOH aufgenommen. Anschließend werden 2,5 ml CHCl₃ hinzugegeben und die Suspension für 1,5 h im Ultraschallbad inkubiert. Die Suspension wird filtriert und der Filter und der abgetrennte Feststoff ein weiteres Mal mit identisch zusammengesetztem Extraktionsmittel für 1 h im Ultraschallbad extrahiert. Zu beiden Extrakten wird für die Phasentrennung 1,25 ml 0,9 % NaCl hinzugegeben. Die organischen Phasen werden kombiniert und bei 45°C im Vakuum getrocknet. Die extrahierten Lipide werden schließlich in einer kleinen Menge Extraktionsmittel aufgenommen und in einem HPLC-Probengefäß über Nacht unter Stickstoffatmosphäre getrocknet. Im Zuge der Analyse der Zellextrakte mittels HPLC kann im Cdc48-psd¹-Stamm eine Erhöhung der produzierten Menge β-Carotin auf 3,7 mg/g Trockengewicht und im Cdc48-psd³-Stamm auf 3,1 mg/g erreicht werden. Dies entspricht 330 % (Cdc48-psd¹) bzw. 280 % (Cdc48-psd³) der β-Carotin Menge in unmodifizierten Kontrollzellen (WT).

Die ^{ΔN}Sic1 -psd³ und Clb2^{ΔDB}-psd³ Stämme produzierten unter den oben genannten Wachstumsbedingungen 150 % (Clb2^{ΔDB}-psd³) bzw. 130 % (^{ΔN}Sic1-psd³) der β-Carotin-Menge der Wildtypzellen. Hierzu wird ebenfalls eine Erhöhung der β-Carotin-abhängigen Fluoreszenz in Abhängigkeit von ^{ΔN}Sic1-psd³ und Clb2^{ΔDB}-psd³ festgestellt: So steigt nach 24 h Inkubation im Dunkeln die Fluoreszenzintensität im ^{ΔN}Sic1-psd³ -Stamm auf 140 % des Wildtypniveaus und im Clb2^{ΔDB}-psd³ -Stamm auf 160 %.

Eine 24 h Inkubation mit mehreren Blaulichtintervallen 420-490 nm (vgl. Cdc48-psd^{1/3}) führt im Npl4-psd¹-Stamm ebenfalls zu einer erhöhten Fluoreszenzintensität auf 180 % im Vergleich zu Zellen ohne weitere Modifikationen.

Weiterhin wird der Effekt einer Zellzyklusarretierung mittels Cdc48-psd³ auf die Produktion von Cordycepin angewendet. Dies zeigt eine sehr starke Steigerung der Produktivität im *CDC48*-*psd³* Stammhintergrund. Bei einer kontinuierlichen 12 h dauernden Beleuchtung mit Blaulicht 420-490 nm (30 µmol/m²s) erreicht dieser Stamm Cordycepin-Titer von 64 µmol pro Liter Kultur und 0,27 mmol/g Trockengewicht, was einer Steigerung von 304 % bzw. 1200 % zum Wildtyp-Stamm entspricht. Die Biomasseanalyse zeigt starke Unterschiede der Biomasse-Zusammensetzung von Wildtyp und zellzyklusarretierten Clb2^{ΔDB}-psd³ Zellen auf. Damit kann gezeigt werden, dass Stoffwechselwege in Hefezellen durch eine optogenetische Zellzyklusregulation gezielt verbessert werden.

### Abbildungen

**Figur 1** zeigt schematisch den geregelten Proteinabbau durch das Ubiquitin-Proteasom-System. Der Proteinabbau durch das Proteasom kann über zwei Wege erfolgen. Die überwiegende Mehrheit der Proteine wird über einen Ubiquitin-abhängigen Mechanismus abgebaut, hierbei wird ein Substrat durch eine enzymatische Kaskade ubiquitinyliert. Diese Kaskade besteht aus einem E1 (Ubiquitin-aktivierendes Enzym), einem E2 (Ubiquitin-konjugierendes Enzym) und einem E3 (Ubiquitin-Protein-Ligase). Das E3 erkennt das Substrat an einer Degradationssequenz (Degron), diese ist zum Abbau hinreichend und auf andere Proteine übertragbar. Das ubiquitinylierte Substrat wird vom Proteasom erkannt, die Proteolyse findet im Inneren des Komplexes statt. Ubiquitin (Ub) wird während des Abbaus durch ubiquitinylierende Enzyme vom Substrat entfernt und dadurch recycelt. Ein zweiter Proteolyseweg ist Ubiquitin-unabhängig. Bestimmte Degrons werden direkt durch das Proteasom erkannt. Die Proteolyse verläuft in der gleichen Weise wie der Ubiquitin-abhängige Abbau im Inneren des Proteasoms.

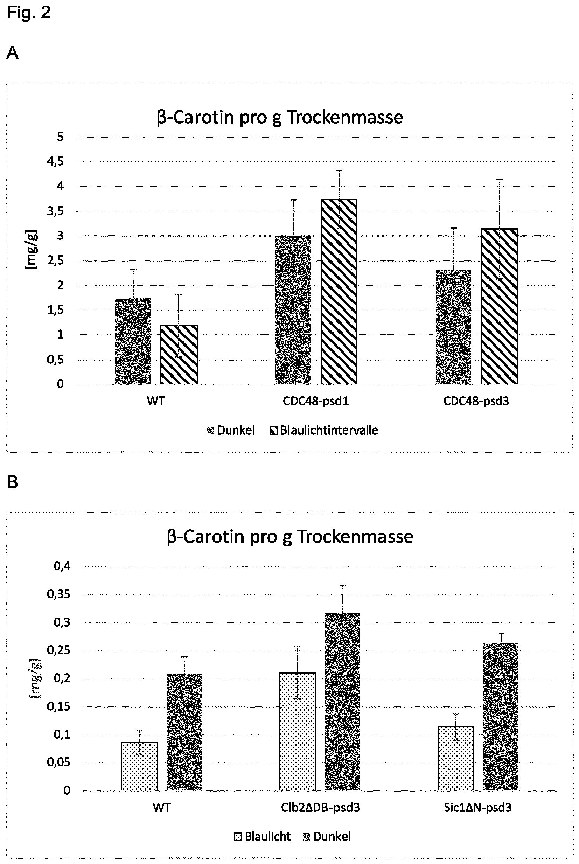

**Figur 2** zeigt die Ergebnisse der erfindungsgemäßen β-Carotin -Extraktion und anschließender Quantifizierung mittels HPLC der verschiedenen Zellzyklus-Arrest-Mutanten.
   **A)** Gezeigt ist die β-Carotin Produktion pro g Trockenmasse in unmodifizierten Kontrollzellen (WT) sowie in Cdc48-psd¹ (CDC48-psd1) und Cdc48-psd³ (CDC48-psd3) Stamm von *Saccharomyces cerevisiae* nach einer Wachstumsphase von 24 h in Dunkelheit und Lichtintervallinkubation (3x 5 h Blaulicht und 3 h Dunkelheit) über einen Zeitraum von 24 h (Blaulichtintervalle) bzw. 24 h Dunkelheit (Dunkel). Durch die Blaulicht-induzierten Stopps des Zellzyklus wird im Cdc48-psd¹ Stamm β-Carotin in Höhe von 3,7 mg pro g Zelltrockenmasse produziert und im Cdc48-psd³-Stamm 3,1 mg pro g Trockenmasse. Dies entspricht 330 % (Cdc48-psd¹) und 280 % (Cdc48-psd³) der β-Carotin Menge die in unmodifizierten Kontrollzellen (WT) produziert werden.
   **B)** Gezeigt ist die β-Carotin Produktion pro g Trockenmasse in unmodifizierten Kontrollzellen (WT) sowie in ^{ΔN}Sic1-psd³ und Clb2^{ΔDB}-psd³ Stämmen. Diese werden durch Akkumulation der Zellzyklusregulatoren ^{ΔN}Sic1-psd³ (Sic1_ΔN) und Clb2^{ΔDB}-psd³ (Clb2_ΔDB) im Dunkeln im Zellzyklus arretiert (12 h Blaulicht-Wachstumsphase gefolgt von 24 h Phase in Dunkelheit (Dunkel) bzw. 24 h Blaulicht (Blaulicht)). Im Falle der Sic1_ΔN und Clb2_ΔDB Stämme wird bei einem kontrollierten Zellzyklus-Stopp Titer von 0,26 mg (Sic1_ΔN) und 0,3 mg (Clb2_ΔDB) pro g Trockengewicht erreicht, was unter diesen Wachstumsbedingungen 150 % (Clb2_ΔDB) und 130 % (Sic1_ΔN) der β-Carotin Menge in unmodifizierten Kontrollzellen (WT) entspricht.

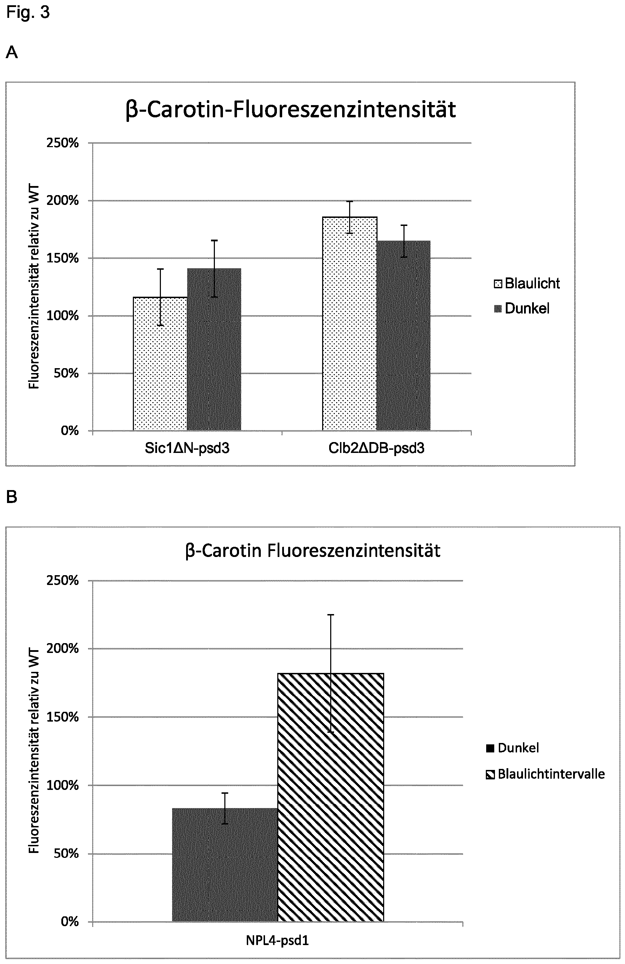

**Figur 3** zeigt die Ergebnisse der Durchflusszytometer-Messung der Fluoreszenz nach β-Carotin in verschiedenen Stämmen.
   **A)** Gezeigt ist die β-Carotin Fluoreszenz in Zellen, mit Clb2^{ΔDB}-psd³ bzw. ^{ΔN}Sic1-psd³, relativ zur Fluoreszenz im Wildtyp (ESM356-1) nach 24 h im Licht bzw. in Dunkelheit. Hierbei wird in Dunkelheit die β-Carotin abhängige Fluoreszenz auf 140 % (Sic1_ΔN) und 160 % (Clb2_ΔDB) des Wildtyp-Niveaus gesteigert.
   **B)** Gezeigt ist die β-Carotin Fluoreszenz im Npl4-psd¹ Stammhintergrund nach 24 h im Dunkeln bzw. nach drei Intervallen mit je 5 h Blaulicht und 3 h Dunkelheit. Unter Blaulicht-Einfluss wird eine Erhöhung der Fluoreszenzwerte auf 180 % der WT-Menge erreicht.

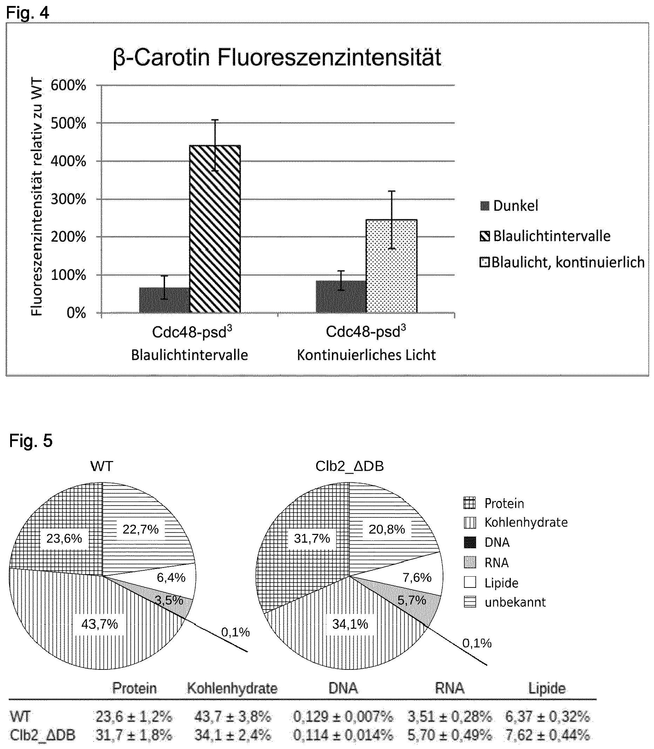

**Figur 4** zeigt die Ergebnisse der Durchflusszytometer-Messung der Fluoreszenz nach β-Carotin im Cdc48-psd³ Stamm. Dargestellt ist der Vergleich zwischen dem Effekt von Blaulichtintervallen und durchgängiger Blaulichtbeleuchtung von Cdc48-psd³ auf die Produktion vom Pflanzeninhaltsstoff β-Carotin anhand der Fluoreszenz im Vergleich zum Wildtyp-Stamm.
**Figur 5** zeigt die Biomassekomposition des ESM356 Wildtyp (WT) und des zellzyklus-arretierten Clb2^{ΔDB}-psd³ (Clb2_ΔDB) Stammes. Dargestellt sind Protein-, Kohlenhydrat-, DNA-, RNA- und Lipidgehalt anteilig an der Zelltrockenmasse.

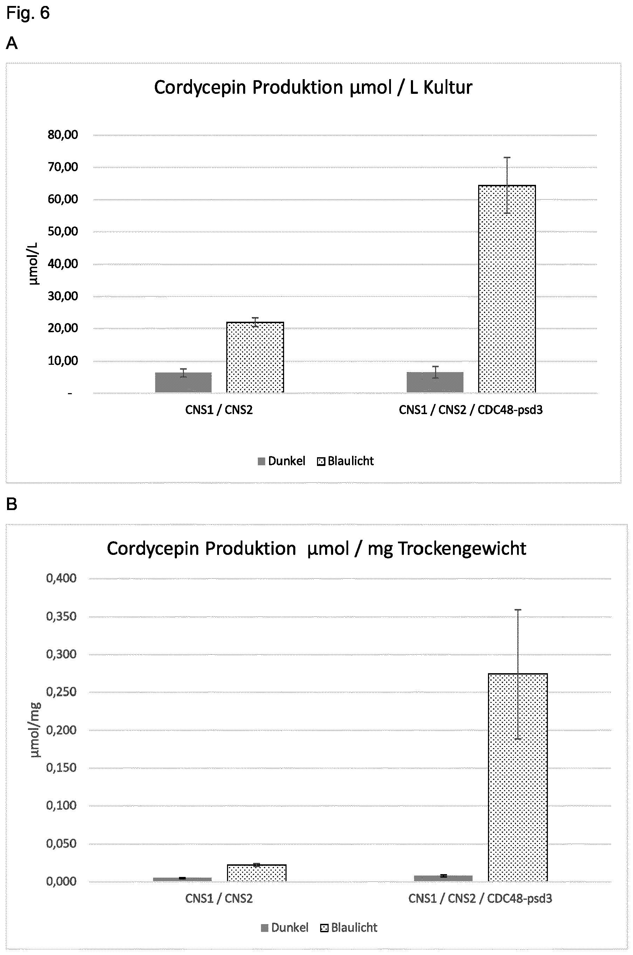

**Figur 6** zeigt die Ergebnisse der Quantifizierung von Cordycepin in einem WT und einem Cdc48-psd³ Stamm. Dargestellt ist die Menge an produziertem Cordycepin nach 12 h Blaulicht-Inkubation in einem WT-Stamm mit Cns1 und Cns2 verglichen mit einem Cdc48-psd³-Stamm mit den notwendigen Enzymen. Dabei kommt es zu einer starken Erhöhung der produzierten Menge Cordycepin pro Liter Kultur im Cdc48-psd³-Stamm mit 64 µmol/l auf 304 % des WT-Niveaus. Relativ zur Trockenmasse ergibt sich das 12fache (0,27 µmol/mg) im Vergleich zum WT.

## Patentansprüche

1. Verfahren zur Herstellung eines pflanzlichen oder pilzlichen Inhaltsstoffes in eukaryotischen Zellen durch optogenetische Kontrolle von mindestens einem Zellzyklusregulator umfassend die Schritte
i) Bereitstellen eines Expressionsvektors, der ein photosensitives Degron aufweist, welches bei Blaulichtexposition zwischen 420-490 nm eine Degradationssequenz freisetzt, welche durch das Ubiquitin-Proteasomen System erkannt wird und wobei der Expressionsvektor für das Protein mindestens eines Zellzyklusregulators kodiert sodass ein Fusionsprotein mit dem photosensitiven Degron entsteht.
ii) Transfektion des Expressionsvektors in eukaryotische Zellen, die den pflanzlichen oder pilzlichen Inhaltsstoff exprimieren, um dessen Produktion durch Blaulichtexposition zwischen 420-490 nm steuerbar zu machen.
iii) Exposition der eukaryotischen Zellen bei Blaulicht zwischen 420-490 nm so dass das Fusionsprotein abgebaut und der pflanzliche oder pilzliche Inhaltsstoff in den eukaryotischen Zellen gesteigert freigesetzt wird.

2. Verfahren zur Herstellung eines pflanzlichen oder pilzlichen Inhaltsstoffes gemäß Anspruch 1, **dadurch gekennzeichnet, dass** der mindestens eine Zellzyklusregulator ausgewählt ist aus der Gruppe Cdc48, Npl4, Sic1 und Clb2.

3. Verfahren zur Herstellung eines pflanzlichen oder pilzlichen Inhaltsstoffes gemäß Anspruch 1 oder 2, **dadurch gekennzeichnet, dass** die eukaryotischen Zellen Hefen sind.

4. Verfahren gemäß einem der Ansprüche 1 bis 3, **dadurch gekennzeichnet, dass** der Pflanzeninhaltsstoff β-Carotin oder Gibberellin ist.

5. Verfahren gemäß Anspruch 4, **dadurch gekennzeichnet, dass** der Pflanzeninhaltsstoff β-Carotin um mindestens 100% gegenüber einem Wildtyp gesteigert wird.

6. Verfahren gemäß Anspruch 4, **dadurch gekennzeichnet, dass** der Pflanzeninhaltsstoff Gibberellin um mindestens 100% gegenüber einem Wildtyp gesteigert wird.

7. Verfahren gemäß einem der Ansprüche 1 bis 3, **dadurch gekennzeichnet, dass** der pilzliche Inhaltsstoff Cordycepin ist.

8. Verfahren gemäß einem der Ansprüche 7, **dadurch gekennzeichnet, dass** der pilzliche Inhaltsstoff Cordycepin um mindestens 100% gegenüber einem Wildtyp gesteigert wird.

9. Verfahren zur Herstellung eines pflanzlichen oder pilzlichen Inhaltsstoffes gemäß einem der vorangegangenen Ansprüche, **dadurch gekennzeichnet, dass** das photosensitive Degron eine Photorezeptordomäne umfasst, wobei eine lichtabhängige strukturelle Änderung eine Degradationssequenz freisetzt zur Erkennung durch das Ubiquitin-Proteasomensystem.

10. Verfahren zur Herstellung eines pflanzlichen oder pilzlichen Inhaltsstoffes gemäß Anspruch 9, **dadurch gekennzeichnet, dass** das photosensitive Degron eine Photorezeptordomäne eines pflanzlichen Phototropins aus der Light Oxygen Voltage (LOV) Familie umfasst.

11. Verfahren zur Herstellung eines pflanzlichen oder pilzlichen Inhaltsstoffes gemäß Anspruch 9, **dadurch gekennzeichnet, dass** die Degradationssequenz die C-terminale Degradationssequenz einer tierischen Ornithindecarboxylase umfasst.
